# EUROPEAN PATENT SPECIFICATION

(11) **EP 1 605 865 B1**
(45) Date of publication and mention of the grant of the patent: **10.12.2008**
(21) Application number: 04757547.7
(22) Date of filing: 16.03.2004
(51) Int. Cl.: A61F 2/01, A61F 2/06, A61L 27/00, A61L 27/56, A61L 27/48, A61L 31/14, A61K 31/12, B32B 3/00, B32B 27/12, A61B 17/12

(54) **STENT WITH THIN FILM COMPOSITE LAMINATE**
STENT MIT LAMINIERTER DÜNNFILMVERBUND
STENT AVEC STRATIFICATION COMPOSITE D'UN FILM MINCE

(30) Priority: 17.03.2003 US 455478 P
(43) Date of publication of application: 21.12.2005
(73) Proprietor: ev3 Endovascular, Inc., Plymouth, MN 55442 (US)
(72) Inventor: KREIDLER, Marc, S., Sunnyvale, CA 94086 (US); DE CICCO, Dino, San Jose, CA 95136 (US); VAN DER BURG, Eric, J., Los Gatos, CA 95032 (US); KUSLEIKA, Richard, Eden Prairie, MN 55346 (US)
(74) Representative: Mallalieu, Catherine Louise
(86) International application number: PCT/US2004/008109
(87) International publication number: WO 2004/082532

(56) References cited:
- WO-A-95/22359
- US-A1- 2002 111 647
- US-A1- 2003 017 775

## Description

### Background of the Invention

### Field of the Invention

The present invention generally relates to thin film composite laminations used as medical devices.

### Description of the Related Art

Embolic stroke is the nation's third leading killer for adults, and is a major cause of disability. There are over 700,000 strokes per year in the United States alone. Of these, roughly 100,000 are hemorragic, and 600,000 are ischemic (either due to vessel narrowing or to embolism). The most common cause of embolic stroke emanating from the heart is thrombus formation due to atrial fibrillation (AF). Approximately 80,000 strokes per year are attributable to atrial fibrillation. Atrial fibrillation is an arrhythmia of the heart that results in a rapid and chaotic heartbeat that produces lower cardiac output and irregular and turbulent blood flow in the vascular system. There are over five million people worldwide with atrial fibrillation, with about four hundred thousand new cases reported each year. Atrial fibrillation is associated with a 500 percent greater risk of stroke due to the condition. A patient with atrial fibrillation typically has a significantly decreased quality of life due, in part, to the fear of a stroke, and the pharmaceutical regimen necessary to reduce that risk.

For patients who develop atrial thrombus from atrial fibrillation, the clot normally occurs in the left atrial appendage (LAA) of the heart. The LAA is a cavity which looks like a small finger or windsock and which is connected to the lateral wall of the left atrium between the mitral valve and the root of the left pulmonary vein. The LAA normally contracts with the rest of the left atrium during a normal heart cycle, thus keeping blood from becoming stagnant therein. However, the LAA often fails to contract with any vigor in patients experiencing atrial fibrillation due to the discoordinate electrical signals associated with AF. As a result, thrombus formation is predisposed to form in the stagnant blood within the LAA.

Blackshear and Odell have reported that of the 1288 patients with non-rheumatic atrial fibrillation involved in their study, 221 (17%) had thrombus detected in the left atrium of the heart. Blackshear JL, Odell JA, Appendage Obliteration to Reduce Stroke in Cardiac Surgical Patients With Atrial Fibrillation, Ann. Thorac. Surg., 1996.61(2):755-9. Of the 221 patients with atrial thrombus, 201 (91%) had the atrial thrombus located within the left atrial appendage. The foregoing suggests that the elimination or containment of thrombus formed within the LAA of patients with atrial fibrillation would significantly reduce the incidence of stroke in those patients.

Pharmacological therapies for stroke prevention, such as oral or systemic administration of warfarin or the like, have been inadequate due to serious side effects of the medications and lack of patient compliance in taking the medication. Invasive surgical or thoracoscopic techniques have been used to obliterate the LAA, however, many patients are not suitable candidates for such surgical procedures due to a compromised condition or having previously undergone cardiac surgery. In addition, the perceived risks of even a thoracoscopic surgical procedure often outweigh the potential benefits. See Blackshear and Odell, above. See also Lindsay BD, Obliteration of the Left Atrial Appendage: A Concept Worth Testing, Ann. Thorac. Surg., 1996.61(2):515.

Despite the various efforts in the prior art, there remains a need for a minimally invasive method and associated devices for reducing the risk of thrombus formation in the left atrial appendage.

Semi-permanently and permanently implantable devices may be used to reduce the risk of thrombus formation due to atrial fibrillation, as well as a variety of other medical conditions. One advantage of these devices is their ability to be introduced during minimally invasive procedures through the use of catheters. Catheters having a small cross sectional profile can be used to access remote locations in the patient through the vascular system. The devices delivered with these catheters are generally capable of assuming a compact form that will fit within the catheter. A variety of these devices incorporate flexible permeable or semi-permeable membranes. Accordingly, the membrane materials often have a minimal thickness as well as appropriate anti-thrombogenic properties.

Numerous materials have proven useful in this respect, particularly expanded polytetrafluoroethylene (ePTFE). Some of these materials are difficult to manufacture into medical devices. Accordingly, there remains a need for improved materials that are both biocompatible and easily processed, as well as improved methods of manufacturing medical devices from such biocompatible materials.

In particular, a variety of medical devices, such as the those for occluding the left atrial appendage, vascular grafts, septal defect closure devices, among others, make use of porous fabrics that are attached to supporting structures. Unfortunately, the physical and chemical properties that make the porous fabrics ideal for use in the vascular system also make them difficult to attach to supporting substrates. Porous fabric sheets of ePTFE can be particularly difficult to attach to supporting structures.

Accordingly, there remains a need for improved porous fabrics and methods of constructing porous fabrics, as well as methods for attaching porous fabrics, such as ePTFE, to supporting structures.

A device with the features of the preamble of claim 1 is disclosed in WO 2002/0111647.

### Summary of the Invention

There is provided in accordance with the present invention a laminated stent-graft suitable for implantation within a medical patient, comprising: a frame, a first membrane, having first membrane pores, a second membrane, having second membrane pores, a mesh bonding layer, having a bonding layer open surface area and bonding layer pores, wherein the first membrane and second membrane are at least partially attached to the mesh bonding layer to form a composite membrane, wherein the composite membrane has a composite membrane open surface area in the range between about 10% and about 50%, and wherein the stent is generally cylindrical and is adjustable from a first configuration having a reduced diameter to a second configuration having an expanded diameter, the stent forming a conduit in the second configuration.

In one embodiment, the first membrane comprises ePTFE. In another embodiment, the first membrane has a thickness in the range of from about 0.001 cm (0.0005 inches) to about 0.25 cm (0.10 inches). In yet another embodiment, the first membrane pores comprise an average pore diameter in the range of from about 1 µm to about 200 µm. In another embodiment, the first membrane comprises an internodal distance in the range of from about 10 µm to about 100 µm.

In one embodiment, the bonding layer comprises polyethylene. In another embodiment, the composite membrane has a thickness in the range of from about 0.003 cm (0.001 inches) to about 0.025 cm (0.010 inches). In yet another embodiment, an element of a support structure is disposed between the first membrane and the second membrane. In another embodiment, the composite membrane open surface area is sufficient to facilitate cellular ingrowth and/or cellular attachment. In another embodiment, the composite membrane has an average thickness within the range of about 0.003 cm (0.001 inches) to about 0.025 cm (0.010 inches).

In one embodiment, an element of the frame is disposed between the first membrane and the second membrane. In yet another embodiment, the composite membrane open surface area is sufficient to facilitate cellular ingrowth and/or cellular attachment. In another embodiment, the composite membrane has an average thickness within the range of about 0.003 cm (0.001 inches) to about 0.025 cm (0.010 inches).

There is provided a biocompatible laminate fabric for use in the present invention including a porous first membrane layer, a porous second membrane layer, and an open mesh bonding layer between the first and second membrane layers. The bonding layer holds the first and second membrane layers together by extending into one or more pores of each membrane layer.

In one embodiment, an element of a support structure is disposed between the first and second membrane layers. In another embodiment, the biocompatible laminate fabric retains sufficient porosity to facilitate cellular ingrowth and/or cellular attachment. The biocompatible laminate fabric has an average thickness that in one embodiment is within the range of about 0.003 cm (0.001 inches) to about 0.025 cm (0.010 inches), preferably less than about 0.01 cm (0.005 inches), more preferably less than about 0.008 cm (0.003 inches), in one embodiment is less than about 0.005 cm (0.002 inches), and in yet another embodiment is about 0.0038 cm (0.0015 inches).

In another embodiment, the biocompatible laminate fabric is no more than about three times as thick as the first membrane layer, preferably is no more than about twice as thick as the first membrane layer, is no more than about three times as thick as the second membrane layer, and in another embodiment, is no more than about twice as thick as the second membrane layer.

In another embodiment, the bonding layer has an average thickness before bonding to the first membrane layer within the range of about 0.001 cm (0.0005 inches) to about 0.01 cm (0.005 inches), about 0.002 cm (0.0008 inches) to about 0.01 cm (0.004 inches), about 0.002 cm (0.0009 inches) to about 0.008 cm (0.003 inches), and in another embodiment, about 0.003 cm (0.001 inches) to about 0.005 cm (0.002 inches).

In another embodiment, the bonding layer has an average pore cross section within the range of about 0.01 cm (0.005 inches) to about 0.50 cm (0.200 inches), and in another embodiment, about 0.051 cm (0.020 inches) to about 0.20 cm (0.080 inches). In one embodiment, the bonding layer has an average spacing between adjacent pores within the range of about 0.001 cm (.0005 inches) to about 1.0 cm (0.400 inches). In one embodiment, the bonding layer has an average open surface area within the range of about 10% to about 90, or preferably within the range of about 30% to about 60%.

In one embodiment, the softening point of the bonding layer is within the range of about 38°C (100°F) to about 149°C (300°F), or preferably within the range of about 93°C (200°F) to about 204°C (400°F). In one embodiment, the melting point of the bonding layer is within the range of about 38°C (100°F) to about 149°C (300°F).

In one embodiment, the first membrane layer has an average thickness within the range of about 0.001 cm (0.0005 inches) to about 0.025 cm (0.010 inches), or preferably about 0.003 cm (0.001 inches) to about 0.005 cm (0.002 inches). In one embodiment, the first membrane layer has an average open surface area within the range of about 10% to about 80%, or preferably about 30% to about 60%.

In one embodiment, the softening point of the first membrane layer is within the range of about 66°C (150°F) to about 260°C (500°F), or preferably about 93°C (200°F) to about 204°C (400°F). In yet another embodiment, the melting point of the first membrane layer is within the range of about 66°C (150°F) to about 260°C (500°F), or preferably about 93°C (200°F) to about 204°C (400°F).

In one embodiment, the difference between the softening point of at least one of the first and second membrane layers and the softening point of the bonding layer is within the range of about 4°C (25°F) to about 93°C (200°F), or preferably is within the range of about 10°C (50°F) to about 38°C (100°F).

In one embodiment of the present invention, the bonding layer holds the first and second membrane layers together by extending into one or more pores of each membrane layer.

In one embodiment, an element of the support structure is disposed between the first and second membrane layers. In yet another embodiment, the biocompatible laminate fabric retains sufficient porosity to facilitate cellular ingrowth and/or cellular attachment. In one embodiment, the biocompatible laminate fabric has an average thickness within the range of about 0.003 cm (0.001 inches) to about 0.025 cm (0.010 inches), or preferably less than about 0.008 cm (0.003 inches).

We describe herein a method of making a biocompatible laminate fabric. The method includes heating a first membrane layer, a second membrane layer and an open mesh bonding layer, under pressure, until the bonding layer becomes sufficiently soft that it flows into pores of each of the first and second membrane layers.

In one embodiment, the layers are heated to a temperature that is at least the softening point of the bonding layer but no more than the softening point of the first and second membrane layers. In another embodiment, the layers are heated to a temperature that is within the range of about 38°C (100°F) to about 93°C (200°F). In another embodiment, the layers are heated under a pressure that is sufficient to allow at least some of the bonding layer to flow into at least one of the pores of each of the first and second membrane layers. In another embodiment, the layers are heated under a pressure that is within the range of about 2 x 10⁶ N/m² (30 psi) to about 2 x 10⁷ N/m² (300 psi), and in another embodiment, the layers are heated under pressure for a period of time that is sufficient to allow at least some of the bonding layer to flow into at least one of the pores of each of the first and second membrane layers. In yet another embodiment, the layers are heated under pressure for a period of time that is within the range of about 1 minute to about 5 minutes.

We further describe a method of attaching a biocompatible laminate fabric to a support structure. The method includes the steps of providing an assembly comprising a first membrane layer, a second membrane layer, an open mesh bonding layer, and an element of a support structure disposed between the first and second membrane layers, and heating the assembly under pressure until the bonding layer becomes sufficiently soft that it flows into pores of each of the first and second membrane layers. In one embodiment, the support structure is a component of an implantable medical device.

In accordance with yet another embodiment of the present invention, the bonding layer secures the first membrane layer to the support structure by extending into one or more pores of the membrane layer.

In accordance with yet another embodiment of the present invention, the bonding layer secures the first membrane layer to the support structure by extending around a portion of the support structure and bonding to itself.

Other devices using the same composite membrane as the stent-graft of the invention are described for information.

We describe a method of blocking the passage of embolic material. The method includes providing a medical device having a first membrane, a second membrane, and a mesh bonding layer. The first membrane has first membrane pores, the second membrane has second membrane pores, and the mesh bonding layer has a bonding layer open surface area and bonding layer pores. The first membrane and second membrane are at least partially attached to the mesh bonding layer to form a composite membrane. The method also includes delivering the medical device to a desired treatment location in a patient. The composite membrane has a composite membrane open surface area in the range between about 10% and about 50%, and the composite membrane open surface area permits tissue ingrowth through the composite membrane and across at least one of the first and second membranes. The composite membrane blocks the passage of embolic material across the composite membrane.

In one example, the desired treatment location is a left atrial appendage, a septal defect, or near an occlusion in a vessel.

Further features and advantages of the present invention will become apparent to those of ordinary skill in the art in view of the detailed description of preferred embodiments which follows, when considered together with the attached drawings and claims.

### Brief Description of the Drawings

FIG. 1 is a perspective view of an occlusion device.
FIG. 2 is a side elevational view of the occlusion device shown in FIG. 1.
FIG. 3 is a perspective view of an alternate example of the occlusion device.
FIG. 4 is a side elevational view of the example shown in FIG. 3.
FIG. 5 is a perspective view of a further example of the occlusion device.
FIG. 6 is a side elevational view of the example of FIG. 5.
FIG. 7A is a side elevational view of the device of FIG. 7.
FIG. 7B is an end view taken along the line 7B-7B of FIG. 7A.
FIG. 8 is a schematic illustration of an inflatable balloon positioned within the occlusion device of FIG. 7.
FIG. 9 is schematic view of a pull string deployment example of the occlusion device of FIG. 7.
FIGS. 10 and 11 are side elevational schematic representations of partial and complete barrier layers on the occlusion device of FIG. 7.
FIG. 12 is a side elevational schematic view of an alternate occlusion device.
FIG. 13 is a schematic view of a bonding layer mesh for use in forming a composite barrier membrane in accordance with one embodiment of the present invention.
FIG. 14 is an exploded cross sectional view of the components of a composite barrier member in accordance with one embodiment of the present invention.
FIG. 15 is a cross sectional view through a composite barrier formed from the components illustrated in FIG. 14.
FIG. 16 is a top plan view of the composite barrier illustrated in FIG. 15.
FIG. 17 is a schematic view of an implant and a deployment system.
FIG. 17A is an enlarged view of a releasable lock in an engaged configuration.
FIG. 17B is an enlarged view as in FIG. 17A, with the core axially retracted to release the implant.
FIG. 18 is a perspective view of a flexible guide tube for use in the configurations of FIG. 17 and/or FIG. 19.
FIG. 19 is a schematic view of an alternate deployment system.
FIGS. 19A - 19B illustrate alternate rotational interlock structures between the deployment catheter and the implant.
FIG. 20 is a schematic cross sectional view through the distal end of a retrieval catheter having an occlusion device removably connected thereto.
FIG. 20A is a side elevational schematic view of the system illustrated in FIG. 20 prior to retraction of the occlusion device into the catheter.
FIG. 20B is a side elevational schematic view as in FIG. 20A with the occlusion device axially elongated and radially reduced and partially drawn into the catheter.
FIG. 20C is a schematic view as in FIG. 20B, with the occlusion device and catheter drawn into a transeptal sheath.
FIG. 21 is a schematic view of a deployment system delivering an implantable containment device to the left atrial appendage;
FIG. 22 is a schematic cross sectional view of an implantable containment device built in accordance with one embodiment of the present invention;
FIG. 23 is a schematic view of a delivery system.
FIG. 23A is a cross sectional view of a deployment catheter as shown in FIG. 23, taken along cut line 23A-23A.
FIG. 24 is a schematic view of the delivery system of FIG. 23, shown attached to an implantable containment device;
FIG. 25A and 25B are a schematic cross sectional view and an end view, respectively, of a loading collar used in the system of FIG. 23;

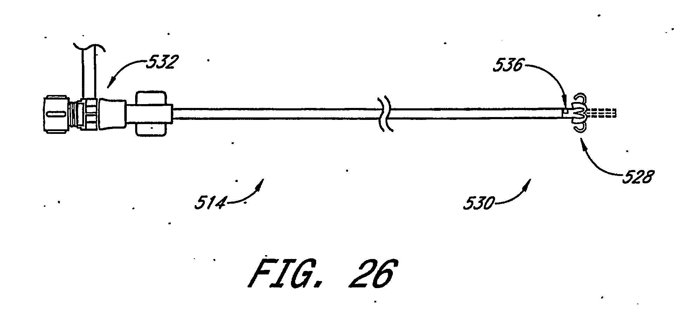

FIG. 26 is a schematic view of a recapture sheath used in the system of FIG. 23;
FIG. 27 is an enlarged partial cross sectional view of the deployment system of FIG. 23;
FIG. 28 is a partial cross sectional view of an axially moveable core used in the system of FIG. 23;
FIG. 28A is a cross sectional view of the axially moveable core of FIG. 28 taken along cut line 28A-28A;
FIG. 29A-C are a schematic view of a transseptal sheath used in combination with the system of FIG. 23;
FIG. 30 is a composite lamination stent in accordance with one embodiment of the present invention;
FIG. 30A is a cross sectional view of the composite lamination stent of FIG. 30 taken along cut line 30A-30A;
FIG. 31 is a composite lamination septal defect closure device shown across a septal wall;
FIG. 31A is an occluder panel of the composite lamination septal defect closure device of FIG. 31;
FIGS. 31B-D are layers of the occluder panel of FIG. 31A; and
FIG. 32 is a composite lamination embolic protection device.

### Detailed Description of the Preferred Embodiments

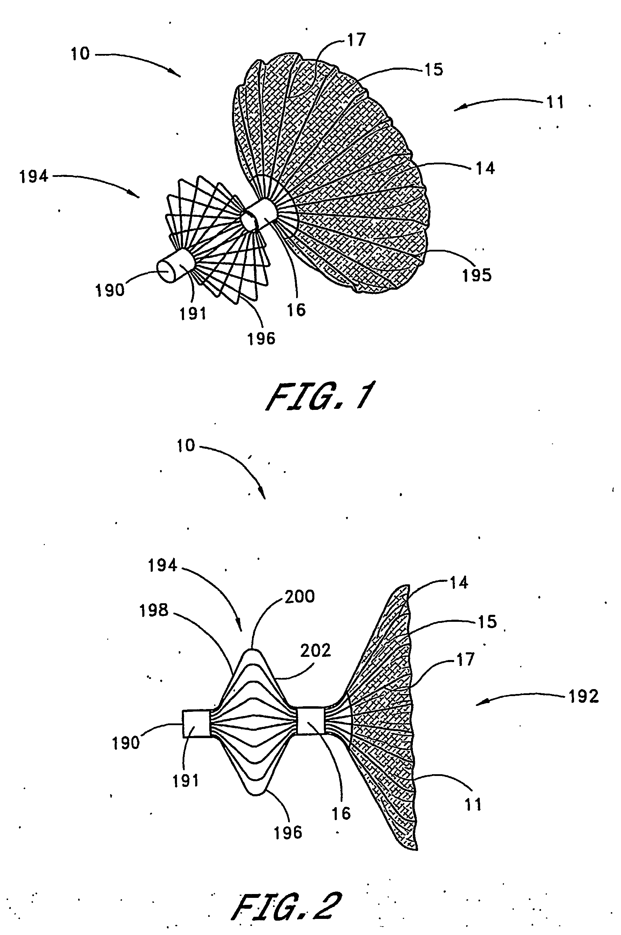

Referring to **FIGS. 1 and 2****,** there is illustrated an occlusion device 10 which is not part of the invention. The term "occlusion" as used herein is intended to include devices that block the passage of embolic material of size which is a function of the pore size of the fabric or other membrane, although the device may at least initially permit the passage of whole blood or blood components. The term "occlusion" as used herein is also intended to include devices that contain embolic material within a volume bounded on at least one side by at least one surface of such device.

In addition, a thin film composite lamination may be used on or to form portions or all of any of a variety of medical devices. Such medical devices include, but are not limited to, vascular implants such as aortic, coronary, peripheral or neurovascular grafts or stent grafts (such as the stent-graft of claim 1 of the invention), occlusion devices such as atrial septal defect (ASD), patent foramen ovale (PFO), ventricular septal defect (VSD) and patent ductus arteriosus (PDA) closure devices, arterial and venous occlusion devices, valve prostheses including the annulus, struts and leaflets, annuloplasty rings, tissue patches, ventricular volume reduction structures, or coverings on any of the foregoing, or other devices as will be appreciated by those of skill in the art.

As shown in FIGS. 1 and 2, the occlusion device 10 comprises an occluding member 11 comprising a frame 14 and a barrier 15. The frame 14 comprises a plurality of radially outwardly extending spokes 17 each having a length within the range of from about 0.5 cm to about 2 cm from a hub 16. In one example, the spokes have an axial length of about 1.5 cm. Depending upon the desired introduction crossing profile of the collapsed occlusion device 10, as well as structural strength requirements in the deployed device, anywhere within the range of from about 3 spokes to about 40 spokes may be utilized. In some examples, anywhere from about 12 to about 24 spokes are utilized and, 18 spokes are utilized in one example.

The spokes are advanceable from a generally axially extending orientation, such as to fit within a tubular introduction catheter, to a radially inclined orientation, as illustrated in FIG. 1 and FIG. 2, following deployment from the catheter. In a self-expandable example, the spokes are biased radially outwardly such that the occlusion member expands to its enlarged, implantation cross-section under its own bias following deployment from the catheter. Alternatively, the occlusion member may be enlarged using any of a variety of enlargement structures such as an inflatable balloon, or a catheter for axially shortening the occlusion member, as is discussed further below.

Preferably, the spokes comprise a metal such as stainless steel, Nitinol, Elgiloy, or others which can be determined through routine experimentation by those of skill in the art. Wires having a circular or rectangular cross-section may be utilized depending upon the manufacturing technique. In one embodiment, rectangular cross section spokes are cut such as by known laser cutting techniques from tube stock, a portion of which forms the hub 16.

The barrier 15 may comprise any of a variety of materials, particularly those which facilitate cellular in-growth, such as ePTFE. The suitability of alternate materials for barrier 15 can be determined through routine experimentation by those of skill in the art. The barrier 15 may be provided on either one or both axially facing sides of the occlusion member. In one embodiment, the barrier 15 comprises two layers, with one layer on each side of the frame 14. The two layers may be bonded to each other around the spokes 17 in any of a variety of ways, such as by heat bonding with or without an intermediate bonding layer such as polyethylene or FEP, adhesives, sutures, clips or staples, and other techniques which will be apparent to those of skill in the art in view of the disclosure herein. In the LAA occlusion device application of the invention, the barrier 15 preferably has a thickness of no more than about 0.008 cm (0.003 inches) and a pore size within the range of from about 5 µm to about 60 µm.

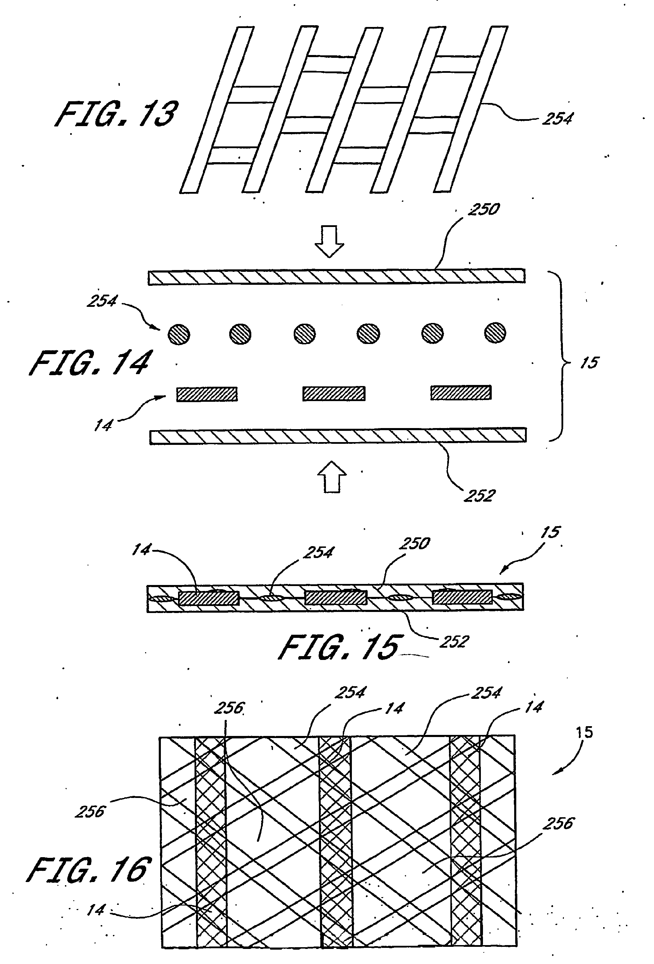

The barrier 15 in one example is securely attached to the frame 14 and retains a sufficient porosity to facilitate cellular ingrowth and/or attachment. One method of manufacturing a suitable composite membrane barrier 15 is illustrated in FIGS. 13-16. The barrier 15 includes a first layer or membrane 250 on a first side of the frame 14, a second layer or membrane 252 on a second side of the frame, and a bonding layer 254 on either of the first side or the second side (shown in Fig. 14 as being on the first side). It will be appreciated that the barrier described herein can be used with any of the devices described herein.

As illustrated schematically in **FIG. 13****,** a bonding layer 254 preferably comprises a mesh or other porous structure having an open surface area within the range of from about 10% to about 90%. The open surface area is the percentage of the layer's or membrane's area that is open to both sides. The term "pores" is intended to have its ordinary meaning as well as a volume within a material through which a gas or liquid may pass. In one embodiment pores have regular shapes, such as cylinders, but in other embodiments, pores have highly irregular shapes, and comprise the open spaces between the fibers of fibrous materials, for example, expanded polytetrafluoroethylene. In another embodiment, the term pores includes the irregularly shaped volumes of open spaces between the fibers of overlapping fibrous materials which are either in contact, or separated by one or more porous intermediate layers, such as an open mesh bonding layer, as described in greater detail below.

The open surface area may be measure using methods well known to those of skill in the art. For example, in one embodiment, the membrane surface is photographed, imaged, or otherwize visualized, and the open surface area is calculated by dividing the sum of the area of the pores by area of the membrane surface visualized. In one embodiment, such calculations are performed by a computer system, signal processing system, and/or software program. Generally, the open surface area of the mesh is within the range of from about 20% to about 70%, often from about 30% to about 60%, an in some applications, from about 40% to about 50% depending on the desired clinical performance. The optimum percentage of open surface area can be determined for any particular application and for any particular material or combination of materials through routine experimentation by one or ordinary skill in the art based on the disclosures herein.

The average pore diameter of the bonding layer 254 is preferably within the range of from about 0.01 cm (0.005 inches)to about 0.13 cm (0.050 inches), often from about 0.025 cm (0.010 inches) to about 0.10 cm (0.040 inches), and in some applications from about 0.038 cm (0.015 inches) to about 0.076 cm (0.030 inches) depending on the desired clinical performance, and, in one embodiment, is about 0.051 cm (0.020 inches). In those embodiments where the pore shape is substantially circular, the diameter is measured in the conventional way. In those embodiments where the pore shape is substantially in the shape of a square, parallelogram, rectangle or other polygon or irregular shape, the diameter is the average of the distance between opposite vertices (sides). For irregular shapes, the pore diameter is the diameter of a circle having an equivalent area to the area of the actual pore. The optimum average pore diameter can be determined for any particular application and for any particular material or combination of materials through routine experimentation by one or ordinary skill in the art based on the disclosure herein. In one embodiment, the average pore area of bonding layer 254 is within the range of from about 0.00013 cm² (0.000020 inched) to about 0.016 cm² (0.0025 inches²), often from about 0.00052 cm² (0.000079 inches²) to about 0.010 cm² (0.0016 inches²), and in some applications from about 0.00114 cm² (0.000177 inches²) to about 0.006 cm² (0.0009 inches²), depending on the desired clinical performance, and, in one embodiment, is about 0.006 cm² (0.0004 inches²). The optimum average pore area can be determined for any particular application and for any particular material or combination of materials through routine experimentation by one or ordinary skill in the art based on the disclosure herein.

As illustrated schematically in FIG. 13, one structure for a bonding layer 254 is a mesh comprising a first plurality of parallel elements or fibers extending in a first direction, and a second plurality of parallel fibers extending in a second direction which is generally transverse to the first direction. The result is to produce a plurality of predictable pores or openings as has been discussed. In one embodiment, the bonding layer 254 includes one or more bonding strips arranged in a woven or an intersecting pattern. In another embodiment, the bonding layer 254 includes a sheet of bonding material with windows or spaces where bonding material is not present. Alternatively, the mesh may comprise a first plurality of elements extending generally parallel to each other and spaced apart, without a second plurality of transverse elements. The bonding layer may alternatively comprise a bead or element of bonding material which extends across the surface of the membrane in a zig zag or sinusoidal pattern. As a further alternative, the bonding mesh may comprise a bead or element of bonding material which extends in a spiral pattern across the surface of the membrane 250. In any of the foregoing structures, equivalent open to closed surface areas ratios can be accomplished, as will be apparent in view of the disclosure herein.

The thickness of the bonding layer 254 can be varied widely, and is generally within the range of from about 0.001 cm (0.0005 inches) to about 0.01 cm (0.005 inches), often from about 0.002 cm (0.0008 inches) to about 0.01 cm (0.004 inches), and in some applications from about 0.002 cm (0.0009 inches) to about 0.008 cm (0.003 inches) depending on the desired clinical performance. In one embodiment, the bonding layer 254 has a thickness of about 0.003 cm (0.001 inches) to about 0.005 cm (0.002 inches). The optimum thickness can be determined for any particular application and for any particular material or combination of materials through routine experimentation by one or ordinary skill in the art based on the disclosure herein.

The bonding layer 254 may comprise any of a variety of materials that have either a softening temperature that is no greater than the softening temperature of the layers 250 and 252, or a melting temperature that is no greater than the melting temperature of the layers 250 and 252. One suitable material, when the layers 250 and 252 include ePTFE, is a polyethylene bonding mesh, such as that available from Smith and Nephew under the code SN9. The suitability of alternate materials for barrier 15 can be determined through routine experimentation by those of skill in the art.

Referring to **FIG. 14****,** the bonding layer 254 is preferably placed adjacent one or both sides of a spoke, stent wall, or other frame element 14. The bonding layer 254 and frame 14 layers are then positioned in-between a first membrane 250 and a second membrane 252 to provide a composite membrane stack. The first membrane 250 and second membrane 252 may comprise any of a variety of materials and thicknesses, depending upon the desired functional result. The membranes 250 and 252 may comprise either the same or different materials, particularly those that facilitate cellular in-growth in a device such as an LAA occlusion device.

In an alternate construction, only a single membrane 250 is used. The membrane 250 is secured to a bonding layer 254. The resulting two layer stack is positioned adjacent a support structure such as struts on an implantable device. The bonding layer 254 is attached to the support structure in any of a variety of ways, such as by melting the bonding layer 254 into a porous surface on the support, or bonding the bonding layer 254 to a compatible surface such as a polymeric support structure, a ceramic structure, or a metallic structure having a compatible bonding layer thereon. Alternatively, the membrane 250 having a bonding layer 254 thereon may be positioned with the bonding layer 254 against a support structure. The membrane and bonding layer laminate may then be wrapped or pinched around a strut or other portion of the support structure, such that the bonding layer 254 surrounds at least a portion of a strut or other support component and is bonded to itself.

In general, the materials that may be included in the bonding layer 254 and/or the membranes 250, 252 for an embodiment of the present invention will depend upon the desired performance of the device (e.g., antigenicity, thrombogenicity, immunogenicity), physical characteristics (e.g., thickness, flexibility, elasticity, porosity), and manufacturing considerations (e.g., bondability, extrudability), as will be apparent to those of skill in the art. Materials that may be used alone or in combination, include: Acrylonitrile, Acrylonitrile Butadiene, Acrylonitrile Butadiene Acrylate, Acrylonitrile Butadiene Styrene, Acrylonitrile Ethylene Styrene, Acrylonitrile Methyl Methacrylate, Acrylonitrile Styrene Acrylate, Cellulose Acetate, Cellulose Acetate Butyrate, Cellulose Acetate Propionate, Cellulose Formaldehyde, Caroxymethyl Cellulose, Cellulose Nitrate, Nitrocellulose Cellulose Propionate, Chlorinated Polyethylene, Chlorinated Polyvinyl Chloride, Cellulose Triacetate, Ethylene Vinyl Acetate, Ethyl Cellulose, Ethylene Ethyl Acrylate, Ethylene Methacrylic Acid, Ethylene Propylene, Ethylene Propylene Diene, Ethylene Tetrafluorethylene, Furan Formaldehyde, Polybutylene Terephthalate, Melamine Formaldehyde, Polyacrylates, Poly(2-hydroxyethyl methacrylate), Polyamide, Polyarylether, Polyaryletherkeone, Polyamide-imide, Polyacrylonitrile, Polyarylamid (polyaramide), Polyarylsulfone, Polyarylate (Polyarylterephthalate), Polybutene-1, Polybutylene Terephthalate, Polycarbonate, Polycyclohexylene Terephthalate, Polychlorotrifluoroethylene, Poly Dialyl Phthalate, Polydicyclopentadiene, Polyether Block Amide, Polyether-etherketone, Polyethylene, High Density Polyethylene, Low Density Polyethylene, Linear Low Density Polyethylene, Linear Medium Density Polyethylene, Medium Density Polyethylene, Ultra High Molecular Weight Polyethylene, Polyethylene Oxide, Polyetherimide, Polyetherketone, Polyethylene Glycol, Polyethersulfone, Polyethylene Terephthalate, Phenol Formaldehyde, Phenol Furfural, Polyimide, Polyisobuthylene, Polyimidesulfone, Poly Methyl Methacrylate, Poly Methyl Methacrylate Imide, Poly 4-Methylpentene-1, Poly p-Oxybenzoate, Polyformaldehyde, Polypropylene, Poly Phthalamide, T Poly Phenylene Ether, Poly Propylene Oxide, Poly Phenylene Sulfide, Poly Phenylene Sulfone, Polystyrene, Polystyrol, Polysulfone, Polytetrafluoroethylene, Polyurethane, Poly Vinyl Acetate, Poly Vinyl Alcohol, Poly Vinyl Butyl, Poly Vinyl Chloride, Poly Vinyl Chloride Acetate, Poly Vinyldene Chloride, Poly Vinyldene Fluoride, Poly Vinyl Fluoride, Poly Vinyl Formal, Poly Vinyl Carbazole, Poly Vinyl Pyrolidone, Styrene Acrylonitrile, Styrene Butadiene, Silicone, Styrene Maleic Anhydride, Styrene A-Methylstyrene, Urea Formaldehyde, Polyester, Vinyl Chloride Ethylene, Vinyl Chloride Ethylene Methyl Acrylate, Vinyl Chloride Methyl Acrylate, Vinyl Chloride Vinyl Acetate, and Vinyl Chloride Vinylidene Chloride. The optimum selection of material composition can be determined for any particular application and for any desired application (*e.g*., facilitate or inhibit cell growth, affect the permeability of red blood cells, affect the permeability of plasma, etc.) through routine experimentation by one or ordinary skill in the art based on the disclosures herein.

These materials, alone or in combination, may optionally be treated to reduce antigenicity, immunogenicity, and thrombogenicity. In one embodiment, a material is treated with glutaraldehyde, urea, or other chaotropic agents (as disclosed in U.S. Pat. Nos. 5,613,982 and 5,632,778, the entire disclosures of which are incorporated by reference herein), barium sulfate (as disclosed in U.S. Pat. Nos. 5,192,311, 4,787,900, and 5,591,225, the entire disclosures of which are incorporated by reference herein) and/or collagen (as disclosed in U. S. Patent No. 5,037,377, the entire disclosure of which is incorporated by reference herein). In one embodiment, both membranes 250 and 252 comprise porous ePTFE.

The membranes 250 and 252 may have either the same or different thicknesses, and generally have a thickness within the range of from about 0.001 cm (0.0005 inches) to about 0.025 cm (0.010 inches), preferably from about 0.002 cm (0.0007 inches) to about 0.02 cm (0.008 inches), often from about 0.002 cm (0.0008 inches) to about 0.01 cm (0.005 inches), and in certain applications, from about 0.002 cm (0.0009 inches) to about 0.008 cm (0.003 inches). In one embodiment, the membranes 250 and 252 each have a thickness on the order of from about 0.003 cm (0.001 inches) to about 0.005 cm (0.002 inches). The optimum thickness can be determined for any particular application and for any particular material or combination of materials through routine experimentation by one or ordinary skill in the art based on the disclosures herein.

The membranes 250 and 252 may have either the same or different average pore diameters. The average pore diameter is often within the range of from about 1 µm to about 200 µm, preferably within the range of from about 5 µm to about 100 µm, and in some application within the range of from about 10 µm to about 70 µm. The optimum average pore diameter or spacing can be determined for any particular application and for any particular material or combination of materials through routine experimentation by one or ordinary skill in the art based on the disclosures herein.

The membranes 250 and 252 may have either the same or different average spacing between the edges of adjacent pores, and generally have an average internodal distance of from about 10 µm to about 100 µm, or exhibit equivalent cellular migration or ingrowth characteristics as membranes having such internodular distances.

In one embodiment, the composite stack of membranes 250 and 252 and bonding layer 254 is heated under pressure. The composite stack is heated to a temperature that is greater than the softening temperature of the bonding layer 254, and is less than the melting temperature of the membranes 250 and 252. Depending on the materials used, the temperature may be greater than the softening temperature of the bonding layer 254, and less than the softening temperature of the membranes 250 and 252. In some applications the temperature is within the range from about 100° to about 300° Celsius, preferably from about 150°C to about 250°C. In one embodiment, the membranes 250 and 252 comprise ePTFE, the bonding layer 254 comprises polyethylene, and the stack is heated to about 200°C. The optimum temperature can be determined for any particular application and for any particular material or combination of materials through routine experimentation by one or ordinary skill in the art based on the disclosures herein.

The melting temperature of the bonding layer 254 in some embodiments is between about 38°C (100°F) and about 260°C (500°F), preferably between about 93°C (200°F) and about 204°C (400°F), and in one medical device application is about 149°C (300°F). The softening temperature of the bonding layer 254 for many medical device applications is between about 38°C (100°F) and about 121°C (250°F), preferably between about 66°C (150°F) and about 93°C (200°F), and in one medical device application is about 71-88°C (160-190°F). The melting temperature of the membranes 250 and 252 can be the same or different and for many medical device applications is between about 66°C (150°F) and about 260°C (500°F), and preferably between about 93°C (200°F) and about (400°F). The softening temperature of the membranes 250 and 252 can be the same or different and for many medical device applications are between about 38°C (100°F) and about 246°C (475°F), preferably between about 93°C (200°F) and in one medical device application is about 191°C (375°F). In general, the membranes are stable up to at least about 20-25°F higher than the melting temperature of the bonding layer.

The optimum relationship between the melting and softening temperatures of the membranes 250 and 252 and the bonding layer 254 can be determined for any particular application and for any particular material or combination of materials through routine experimentation by one or ordinary skill in the art based on the disclosures herein.

In one embodiment in which the membranes 250 and 252 comprise ePTFE and the bonding layer 254 comprises polyethylene, the composite stack is generally heated for about 1 minute to about 5 minutes, preferably for about 2 minutes to about 4 minutes, and in one embodiment, is heated for about 3 minutes. The composite stack is heated under about 10 x 10⁵ N/m² (15 psi) to about 6.9 x 10⁶ N/m² (1000 psi), preferably from about 1.4 x 10⁵ N/m² (20 psi) to about 3.4 x 10⁶N/m² (500 psi), and in one embodiment, from about 2.1 x 10⁵ N/m² (30 psi) to about 2.1 x 10⁶N/m² (300 psi) to provide a finished composite membrane assembly with an embedded frame 14 as illustrated schematically in **FIG. 15****.** The optimum pressure can be determined for any particular application and for any particular material or combination of materials through routine experimentation by one or ordinary skill in the art based on the disclosures herein.

The foregoing procedure allows the bonding mesh 254 to flow into the pores of the first and second membranes 250 and 252 and gives the composite membrane barrier 15 greater strength (both tensile and tear strength) than the components without the bonding mesh 254. The composite membrane barrier 15 allows uniform bonding while maintaining porosity of the membrane 15, to facilitate tissue attachment. In one embodiment, by flowing a thermoplastic bonding layer into the pores of the outer layers 250 and 252, the composite membrane barrier's flexibility is preserved and the overall composite membrane barrier 15 thickness can be minimized.

The resulting composite membrane barrier 15 generally has a thickness within the range of from about 0.003 cm (0.001 inches) to about 0.025 cm (0.010 inches), often less than about 0.01 cm (0.005 inches), preferably less than about 0.01 cm (0.004 inches) and in one embodiment about 0.008 cm (0.003 inches). The final composite membrane barrier 15 generally has an average pore diameter within the range of from about 1 µm to about 100 µm, often within the range of from about 5µm to about 60µm, and in some applications, equivalent to ePTFE having an internodal distance within the range of from about 30 µm to about 60 µm.

In one embodiment, the composite membrane barrier 15 generally has an average spacing between the edges of adjacent pores within the range of from about 50 µm to about 2,500 µm, often within the range of from about 50 µm to about 1000 µm, preferably within the range of from about 50 µm to about 300 µm, and in one embodiment within the range of from about 75 µm to about 150 µm. The final composite membrane barrier 15 generally has an open surface area of about 10% to about 50%, often about 10% to about 40%, preferably about 10% to 30% and in one embodiment about 35%. The optimum values for each of these physical parameters and the optimum relationship among these various physical parameters can be determined for any particular application and for any particular material or combination of materials through routine experimentation by one or ordinary skill in the art based on the disclosures herein.

The term "composite membrane open surface area" includes the term's ordinary meaning, and also refers to the open surface area of the composite membrane barrier 15. As described above, the composite membrane barrier 15 has two sides, defined by first and second layers 250, 252. The composite membrane open surface area is the percentage of the compose membrane's area that is open to both sides.

As illustrated in the top plan view of **FIG. 16****,** the composite membrane barrier 15 has a plurality of "unbounded" windows, areas or pores 256. The pores 256 are suitable for cellular attachment and/or ingrowth. The windows 256 may be bounded by the frame 14 struts, and the cross-hatch or other wall pattern formed by the bonding layer 254, described further herein. In one embodiment, a regular window 256 pattern is produced in the bonding layer 254. The composite membrane barrier 15 generally allows cellular growth through and across the surface of the membrane barrier 15 within about two weeks after implantation, with endothelialization generally occurring within about one month.

Referring back to **FIGS. 1 and 2****,** the occlusion device 10 may be further provided with a bulking element or stabilizer 194. The stabilizer 194 may be spaced apart along an axis from the occluding member 11. In the illustrated example, a distal end 190 and a proximal end 192 are identified for reference. The designation proximal or distal is not intended to indicate any particular anatomical orientation or deployment orientation within the deployment catheter. As shown in FIGS. 1 and 2, the stabilizer 194 is spaced distally apart from the occluding member 11.

In one example, the occluding member 11 is placed in the left atrial appendage and has an expanded diameter within the range of from about 1 cm to about 5 cm, and in one embodiment, about 3 cm. The axial length of the occluding member 11 in an expanded, unstressed orientation from the distal end 192 to the hub 16 is on the order of about 1 cm. The overall length of the occlusion device 10 from the distal end 192 to the proximal end 190 is within the range of from about 1.5 cm to about 4 cm and, in one example, about 2.5 cm. The axial length of the stabilizer 194 between distal hub 191 and proximal hub 16 is within the range of from about 0.5 cm to about 2 cm, and, in one example, about 1 cm. The expanded diameter of the stabilizer 194 is within the range of from about 0.5 cm to about 2.5 cm, and in one example, about 1.4 cm, The outside diameter of the distal hub 191 and proximal hub 16 is about 2.5 mm.

Preferably, the occlusion device 10 is provided with one or more retention structures for retaining the device in the left atrial appendage or other body cavity or lumen. In the illustrated examples, a plurality of barbs or other anchors 195 are provided for engaging adjacent tissue to retain the occlusion device 10 in its implanted position and to limit relative movement between the tissue and the occlusion device 10. In one example, anchors 195 are provided on one or more of the spokes 17, or other portion of frame 14. In one example, every spoke 17, every second spoke 17, or every third spoke 17 is provided with one or two or more anchors 195 each.

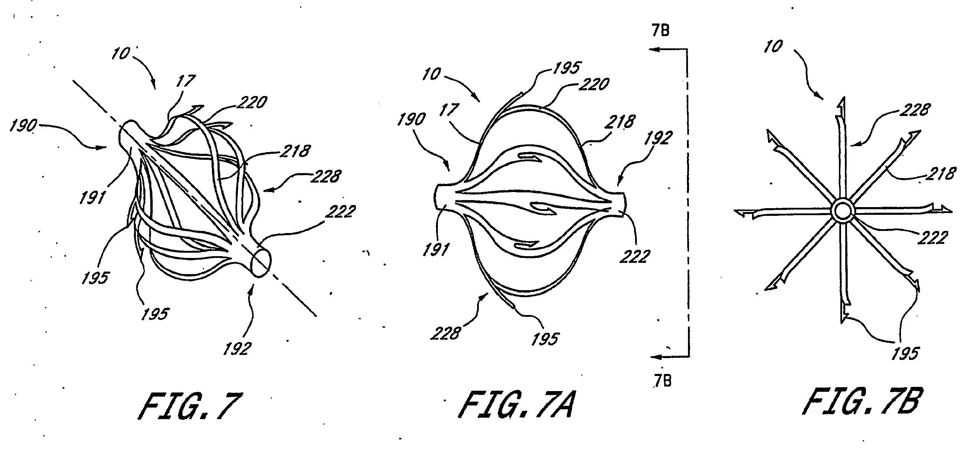

In one example, the anchor 195 is in the form of a barb, with one on each spoke 17 for extending into tissue at or near the opening of the LAA. Depending upon the embodiment, two or three barbs may alternatively be desired on each spoke 17. In the single barb per spoke 17 embodiment, such as that illustrated in FIG. 7, each barb is inclined in a proximal direction. This is to inhibit proximal migration of the implant out of the left atrial appendage. In this context, distal refers to the direction into the left atrial appendage, and proximal refers to the direction from the left atrial appendage into the heart.

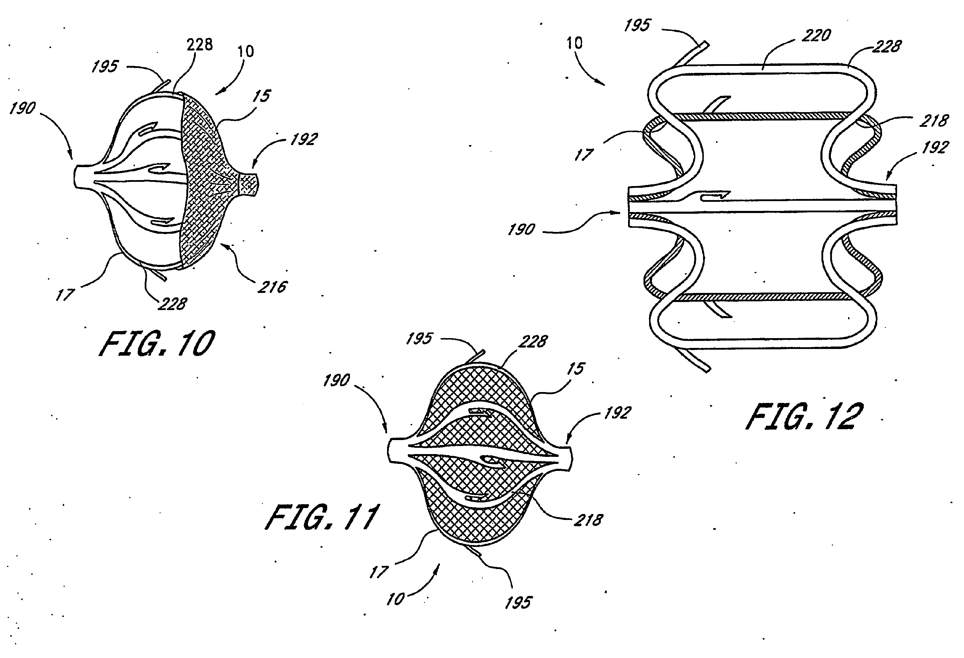

Alternatively, one or more barbs may face distally, to inhibit distal migration of the occlusion device deeper into the LAA. Thus the implant may be provided with at least one proximally facing barb and at least one distally facing barb. For example, in an embodiment of the type illustrated in FIG. 12, discussed below, a proximal plurality of barbs may be inclined in a first direction, and a distal plurality of barbs may be inclined in a second direction (not shown), to anchor the implant against both proximal and distal migration.

One or more anchors 195 may also be provided on the stabilizer 194, such that it assists not only in orienting the occlusion device 10 and resisting compression of the LAA, but also in retaining the occlusion device 10 within the LAA. Any of a wide variety of structures may be utilized for anchor 195, either on the occluding member 11 or the stabilizer 194 or both, such as hooks, barbs, pins, sutures, adhesives, ingrowth surfaces and others which will be apparent to those of skill in the art in view of the disclosure herein.

In one example, the occlusion device 10 is positioned within a tubular anatomical structure to be occluded, such as the left atrial appendage. In a left atrial appendage application, the occluding member 11 is positioned across or near the opening to the LAA and the stabilizer 194, if present, is positioned within the LAA.

The frame 14 is preferably movable between a reduced cross-sectional profile (not shown) for transluminal advancement into the left atrial appendage, and an enlarged cross-sectional orientation as illustrated in FIGS. 1-6 to fill or to substantially fill a cross-section through the LAA. The stabilizing frame may enlarge to a greater cross section than the (pre-stretched) anatomical cavity, to ensure a tight fit and minimize the likelihood of migration or leakage. One convenient construction includes a plurality of elements which are radially outwardly expandable in response to axial compression of a distal hub towards a proximal hub. Elements each comprise a distal segment and a proximal segment connected by a bend. The elements may be provided with a bias in the direction of the radially enlarged orientation as illustrated in FIG. 2, or may be radially expanded by applying an expansion force such as an axially compressive force between a distal portion and a proximal portion or a radial expansion force such as might be applied by an inflatable balloon. The frame elements on the stabilizing member may conveniently be formed by laser cutting the same tube stock as utilized to construct the distal hub 191, proximal hub 16 and frame 14, as will be apparent to those of skill in the art in view of the disclosure herein. Alternatively, the various components of the occlusion device 10 may be separately fabricated or fabricated in subassemblies and secured together during manufacturing.

A radiopaque dye or other visualizable media may be introduced post implantation on one side or the other of the occlusion device, to permit visualization of any escaped blood or other fluid past the occlusion device for any of the occlusion devices disclosed herein. For example, in the context of a left atrial appendage application, the occlusion device may be provided with a central lumen or other capillary tube or aperture which permits introduction of a visualizable dye from the deployment catheter through the occlusion device and into the entrapped space on the distal side of the occlusion device. Alternatively, dye may be introduced into the entrapped space distal to the occlusion device such as by advancing a small gauge needle from the deployment catheter through the barrier 15 on the occlusion device, to introduce dye.

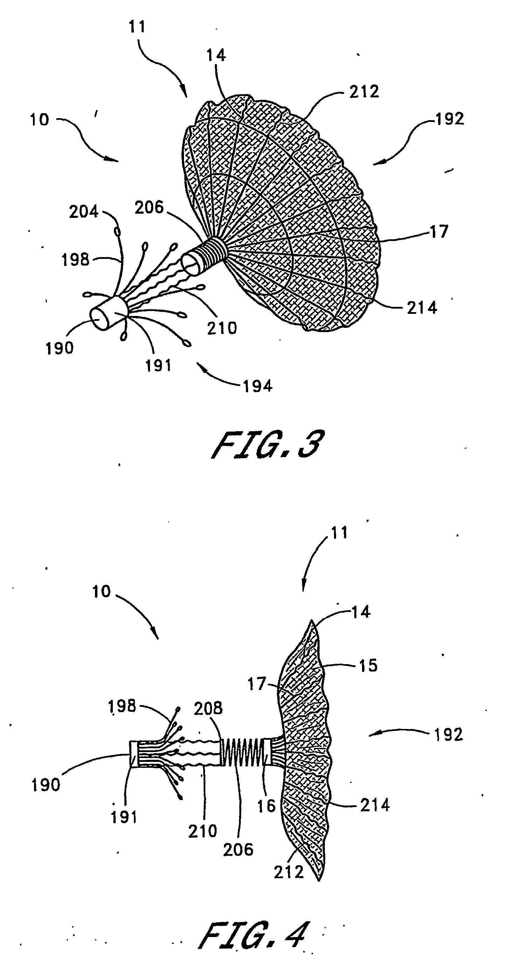

Another occlusion device 10 is illustrated in **FIGS. 3 and 4****.** The occlusion device 10 comprises an occlusion member 11 and a stabilizing member 194 as previously discussed. In this example, however, each of the distal segments 198 inclines radially outwardly in the proximal direction and terminates in a proximal end 204. The proximal end 204 may be provided with an atraumatic configuration, for pressing against, but not penetrating, the wall of the left atrial appendage or other tubular body structure. Three or more distal segments 198 are preferably provided, and generally anywhere within the range of from about 6 to about 20 distal segments 198 may be used. In one embodiment, 9 distal segments 198 are provided. In this example, three of the distal segments 198 have an axial length of about 5 mm, and 6 of the distal segments 198 have an axial length of about 1 cm. Staggering the lengths of the distal segments 198 may axially elongate the zone in the left atrial appendage against which the proximal ends 204 provide anchoring support for the occlusion device 10.

The occlusion device 10 illustrated in FIGS. 3 and 4 includes a hinge 206 to allow the longitudinal axis of the occlusion member 11 to be angularly oriented with respect to the longitudinal axis of the stabilizing member 194. In the illustrated example, the hinge 206 is a helical coil, although any of a variety of hinge structures can be utilized. The illustrated embodiment may be conveniently formed by laser cutting a helical slot through a section of the tube from which the principal structural components of the occlusion device 10 are formed. At the distal end of the hinge 206, an annular band 208 connects the hinge 206 to a plurality of axially extending struts 210. In the illustrated example, three axial struts 210 are provided, spaced equilaterally around the circumference of the body. Axial struts 210 may be formed from a portion of the wall of the original tube stock, which portion is left in its original axial orientation following formation of the distal segments 198 such as by laser cutting from the tubular wall.

The occlusion member 11 is provided with a proximal zone 212 on each of the spokes 17. Proximal zone 212 has an enhanced degree of flexibility, to accommodate the fit between the occlusion member 11 and the wall of the left atrial appendage. Proximal section 212 may be formed by reducing the cross sectional area of each of the spokes 17, which may be provided with a wave pattern as illustrated.

Each of the spokes 17 terminates in a proximal point 214. Proximal point 214 may be contained within layers of the barrier 15, or may extend through or beyond the barrier 15 such as to engage adjacent tissue and assist in retaining the occlusion device 10 at the deployment site.

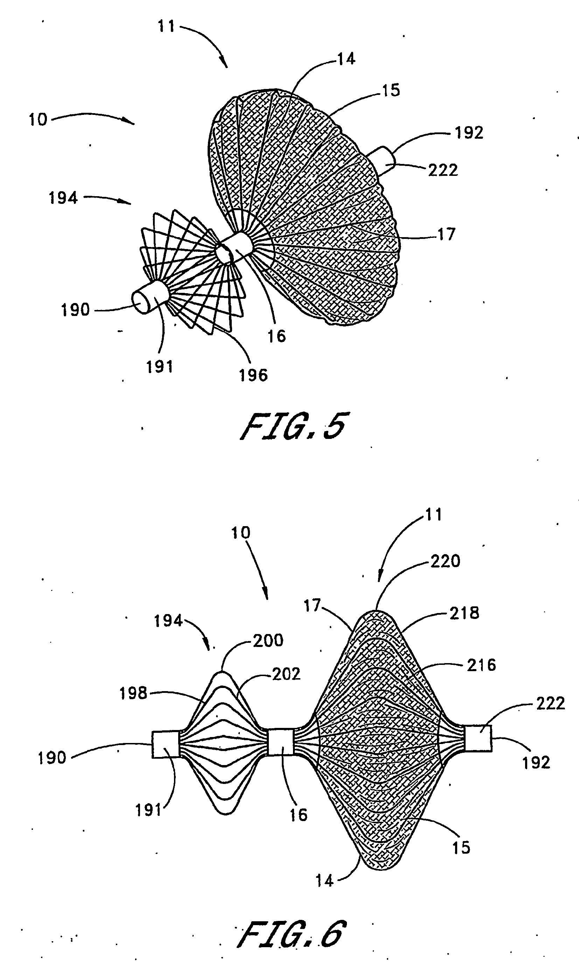

Referring to **FIGS. 5 and 6**, another occlusion device 10 is provided. The occlusion device 10 is provided with a proximal face 216 on the occlusion member 11, instead of the open and proximally concave face on the embodiment of FIGS. 1 and 2. The proximal face 216 is formed by providing a proximal spoke 218 which connects at an apex 220 to some or all of the distal spokes 17. The proximal spoke 218, and corresponding apex 220 and distal spoke 17 may be an integral structure, such as a single ribbon or wire, or element cut from a tube stock as has been discussed.

Proximal spokes 218 are each attached to a hub 222 at the proximal end 192 of the occlusion device 10. The barrier 15 may surround either the proximal face or the distal face or both on the occlusion member 11. In general, provision of a proximal spoke 218 connected by an apex 220 to a distal spoke 17 provides a greater radial force than a distal spoke 17 alone, which will provide an increased resistance to compression if the occlusion member 11 is positioned with the LAA.

Referring to FIGS. 7-12, alternate structures of the occlusion device are illustrated. In general, the occlusion device 10 comprises an occluding member but does not include a distinct stabilizing member as has been illustrated in connection with previous embodiments. Any of the examples previously disclosed herein may also be constructed using the occluding member only, and omitting the stabilizing member as will be apparent to those of skill in the art in view of the disclosure herein.

Referring to **FIG. 7****,** an occluding device 10 comprises a proximal end 192, a distal end 190, and a longitudinal axis extending therebetween. A plurality of supports 228 extend between a proximal hub 222 and a distal hub 191. At least two or three supports 228 are provided, and preferably at least about ten. In one embodiment, sixteen supports 228 are provided. However, the precise number of supports 228 can be modified, depending upon the desired physical properties of the occlusion device 10 as will be apparent to those of skill in the art in view of the disclosure herein.

Each support 228 comprises a proximal spoke portion 218, a distal spoke portion 17, and an apex 220 as has been discussed. Each of the proximal spoke portion 218, distal spoke portion 17 and apex 220 may be a region on an integral support 228, such as a continuous rib or frame member which extends in a generally curved configuration as illustrated with a concavity facing towards the longitudinal axis of the occlusion device 10. Thus, no distinct point or hinge at apex 220 is necessarily provided.

At least some of the supports 228, and in one example, each support 228, is provided with one or two or more barbs 195. In the illustrated configuration, the occlusion device 10 is in its enlarged orientation, such as for occluding a left atrial appendage or other body cavity or lumen. In this orientation, each of the barbs 195 projects generally radially outwardly from the longitudinal axis, and is inclined in the proximal direction. One or more barbs may also be inclined distally, as is discussed elsewhere herein. In an example where the barbs 195 and corresponding support 228 are cut from a single ribbon, sheet or tube stock, the barb 195 will incline radially outwardly at approximately a tangent to the curve formed by the support 228.

The occlusion device 10 constructed from the frame illustrated in FIG. 7 may be constructed in any of a variety of ways, as will become apparent to those of skill in the art in view of the disclosure herein. In one method, the occlusion device 10 is constructed by laser cutting a piece of tube stock to provide a plurality of axially extending slots in-between adjacent supports 228. Similarly, each barb 195 can be laser cut from the corresponding support 228 or space in-between adjacent supports 228. The generally axially extending slots which separate adjacent supports 228 end a sufficient distance from each of the proximal end 192 and distal end 190 to leave a proximal hub 222 and a distal hub 191 to which each of the supports 228 will attach. In this manner, an integral cage structure may be formed. Alternatively, each of the components of the cage structure may be separately formed and attached together such as through soldering, brazing, heat bonding, adhesives, and other fastening techniques which are known in the art. A further method of manufacturing the occlusion device 10 is to laser cut a slot pattern on a flat sheet of appropriate material, such as a flexible metal or polymer, as has been discussed in connection with previous embodiments. The flat sheet may thereafter be rolled about an axis and opposing edges bonded together to form a tubular structure.

The apex portion 220 which carries the barb 195 may be advanced from a low profile orientation in which each of the supports 228 extend generally parallel to the longitudinal axis (not shown), to an implanted orientation as illustrated, in which the apex 220 and the barb 195 are positioned radially outwardly from the longitudinal axis. The support 228 may be biased towards the enlarged orientation, or may be advanced to the enlarged orientation under positive force following positioning within the tubular anatomical structure, in any of a variety of manners.

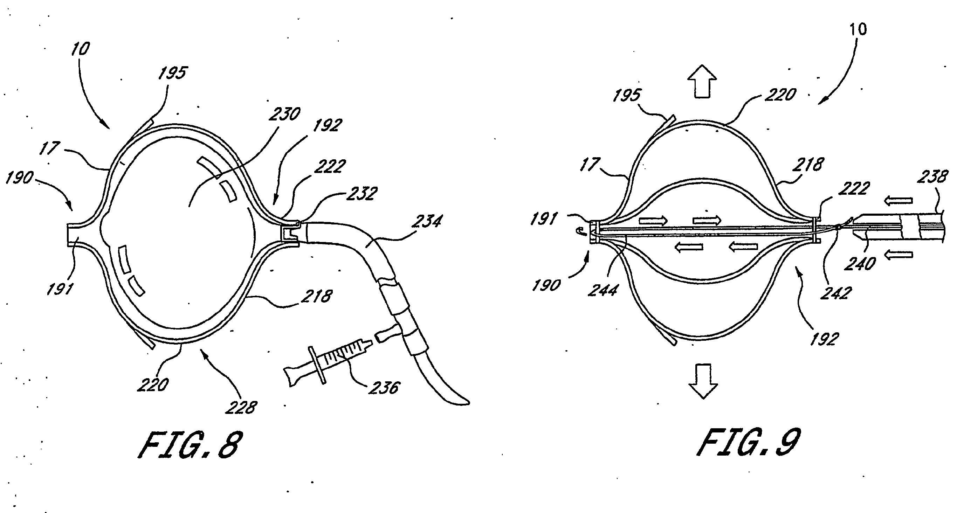

In one example, positive force is used to advance the support 228 to an enlarged orientation. Referring to **FIG. 8****,** an inflatable balloon 230 is positioned within the occlusion device 10. Inflatable balloon 230 is connected by way of a removable coupling 232 to an inflation catheter 234. Inflation catheter 234 is provided with an inflation lumen for providing communication between an inflation media source 236 outside of the patient and the balloon 230. Following positioning within the target body lumen, the balloon 230 is inflated, thereby engaging barbs 195 with the surrounding tissue. The inflation catheter 234 is removed by decoupling the removable coupling 232. The balloon 230 may be either left in place within the occlusion device 10, or deflated and removed by the inflation catheter 234.

In an alternate embodiment, the supports 228 are radially enlarged such as through the use of a deployment catheter 238, as shown, for example, in **FIG. 9****.** Deployment catheter 238 comprises a lumen for movably receiving a deployment element such as a flexible line 240. Deployment line 240 extends in a loop 244 formed by an aperture or slip knot 242. As will be apparent from FIG. 9, proximal retraction on the deployment line 240 while resisting proximal movement of proximal hub 222 such as by using the distal end of the catheter 238 will cause the distal hub 191 to be drawn towards the proximal hub 222, thereby radially enlarging the cross-sectional area of the occlusion device 10. Depending upon the material utilized for the occlusion device 10, the supports 228 will retain the radially enlarged orientation by elastic deformation, or may be retained in the enlarged orientation such as by securing the slip knot 242 immovably to the deployment line 240 at the fully radially enlarged orientation. This may be accomplished in any of a variety of ways, using additional knots, clips, adhesives, or other techniques known in the art.

A variety of alternative structures may be utilized, to open or enlarge the occlusion device 10 under positive force. For example, Referring to FIG. 9, a pullwire 240 may be removably attached to the distal hub 191 or other distal point of attachment on the occlusion device 10. Proximal retraction of the pullwire 240 while resisting proximal motion of the proximal hub 222 such as by using the distal end of the catheter 238 will cause enlargement of the occlusion device 10 as has been discussed. The pullwire 240 may then be locked with respect to the proximal hub 222 and severed or otherwise detached to enable removal of the deployment catheter 238 and proximal extension of the pullwire 240. Locking of the pullwire with respect to the proximal hub 222 may be accomplished in any of a variety of ways, such as by using interference fit or friction fit structures, adhesives, a knot or other technique depending upon the desired catheter design.

Referring to **FIGS. 10 and 11****,** the occlusion device 10 may be provided with a barrier 15 such as a mesh or fabric as has been previously discussed. Barrier 15 may be provided on only one hemisphere such as proximal face 216, or may be carried by the entire occlusion device 10 from proximal end 192 to distal end 190. The barrier may be secured to the radially inwardly facing surface of the supports 228, as illustrated in FIG. 11, or may be provided on the radially outwardly facing surfaces of supports 228, or both.

A further example of the occlusion device 10 is illustrated in **FIG. 12****,** in which the apex 220 is elongated in an axial direction to provide additional contact area between the occlusion device 10 and the wall of the tubular structure. In this embodiment, one or two or three or more anchors 195 may be provided on each support 228, depending upon the desired clinical performance. The occlusion device 10 illustrated in FIG. 12 may also be provided with any of a variety of other features discussed herein, such as a partial or complete barrier 15 (not shown). In addition, the occlusion device 10 illustrated in FIG. 12 may be enlarged using any of the techniques disclosed elsewhere herein.

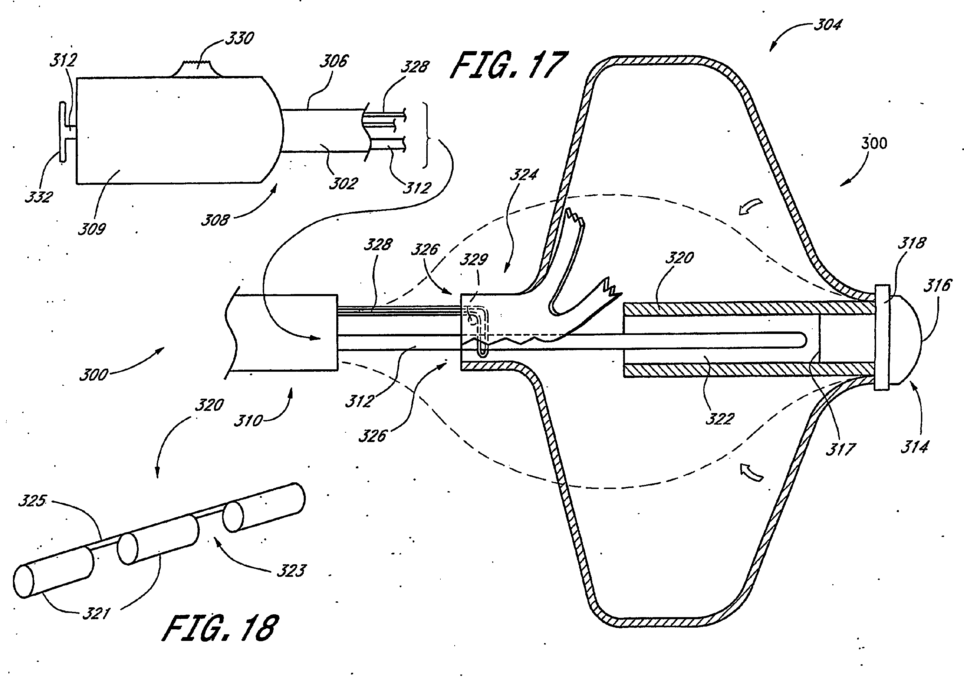

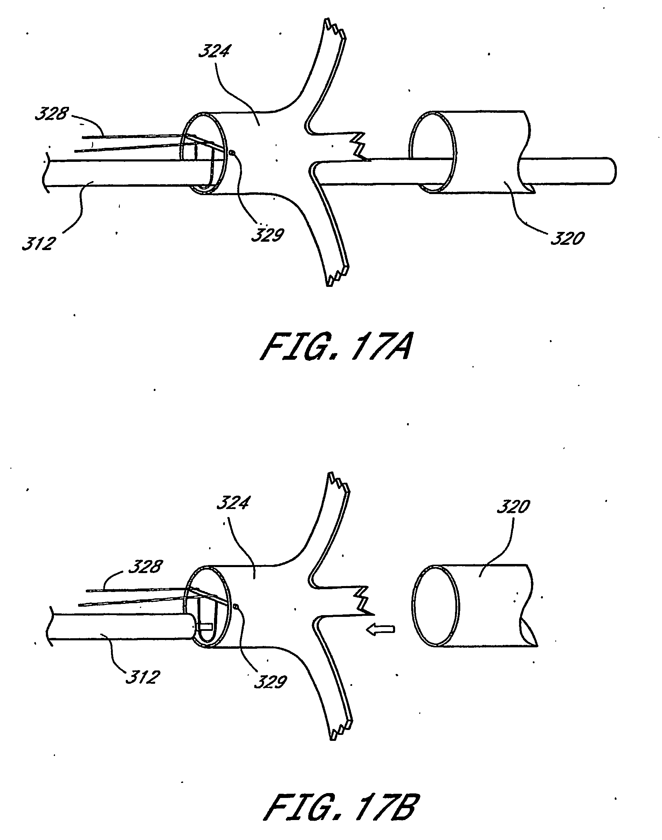

Referring to **FIG. 17****,** there is schematically illustrated an adjustable implant deployment system 300 which comprises generally a catheter 302 for placing a detachable implant 304 within a body cavity or lumen, as has been discussed. The catheter 302 comprises an elongate flexible tubular body 306, extending between a proximal end 308 and a distal end 310. The catheter is shown in highly schematic form, for the purpose of illustrating the functional aspects thereof. The catheter body will have a sufficient length and diameter to permit percutaneous entry into the vascular system, and transluminal advancement through the vascular system to the desired deployment site. For example, in an example intended for access at the femoral artery and deployment within the left atrial appendage, the catheter 302 will have a length within the range of from about 50 cm to about 150 cm, and a diameter of generally no more than about 15 French. Further dimensions and physical characteristics of catheters for navigation to particular sites within the body are well understood in the art and will not be further described herein.

The tubular body 306 is further provided with a handle 309 generally on the proximal end 308 of the catheter 302. The handle 309 permits manipulation of the various aspects of the implant deployment system 300, as will be discussed below. Handle 309 may be manufactured in any of a variety of ways, typically by injection molding or otherwise forming a handpiece for single-hand operation, using materials and construction techniques well known in the medical device arts.

The implant 304 may be in the form of any of those described previously herein, as modified below. In general, the implant is movable from a reduced crossing profile to an enlarged crossing profile, such that it may be positioned within a body structure and advanced from its reduced to its enlarged crossing profile to obstruct bloodflow or perform other functions while anchored therein. The implant 304 may be biased in the direction of the enlarged crossing profile, may be neutrally biased or may be biased in the direction of the reduced crossing profile. Any modifications to the device and deployment system to accommodate these various aspects of the implant 304 may be readily accomplished by those of skill in the art in view of the disclosure herein.

In the illustrated example, the distal end 314 of the implant 304 is provided with an implant plug 316. Implant plug 316 provides a stopping surface 317 for contacting an axially movable core 312. The core 312 extends axially throughout the length of the catheter body 302, and is attached at its proximal end to a core control 332 on the handle 309.'

The core 312 may comprise any of a variety of structures which has sufficient lateral flexibility to permit navigation of the vascular system, and sufficient axial column strength to enable reduction of the implant 304 to its reduced crossing profile. Any of a variety of structures such as hypotube, solid core wire, "bottomed out" coil spring structures, or combinations thereof may be used, depending upon the desired performance of the finished device. In one embodiment, the core 312 comprises stainless steel tubing.

The distal end of core 312 is positioned within a recess or lumen 322 defined by a proximally extending guide tube 320. In the illustrated example, the guide tube 320 is a section of tubing such as metal hypotube, which is attached at the distal end 314 of the implant and extends proximally within the implant 304. The guide tube 320 preferably extends a sufficient distance in the proximal direction to inhibit buckling or prolapse of the core 312 when distal pressure is applied to the core control 332 to reduce the profile of the implant 304. However, the guide tube 320 should not extend proximally a sufficient distance to interfere with the opening of the implant 304.

As will be appreciated by reference to FIG. 17, the guide tube 320 may operate as a limit on distal axial advancement of the proximal end 324 of implant 304. Thus, the guide tube 320 preferably does not extend sufficiently far proximally from the distal end 314 to interfere with optimal opening of the implant 304. The specific dimensions are therefore relative, and will be optimized to suit a particular intended application. In one example, the implant 304 has an implanted outside diameter within the range of from about 5 mm to about 45 mm, and an axial implanted length within the range of from about 5 mm to about 45 mm. The guide tube 320 has an overall length of about 3 mm to about 35 mm, and an outside diameter of about 0.24 cm (0.095 inches).

An alternate guide tube 320 is schematically illustrated in **FIG. 18****.** In this configuration, the guide tube 320 comprises a plurality of tubular segments 321 spaced apart by an intervening space 323. This allows increased flexibility of the guide tube 320, which may be desirable during the implantation step, while retaining the ability of the guide tube 320 to maintain linearity of the core 312 while under axial pressure. Although three segments 321 are illustrated in FIG. 18, as many as 10 or 20 or more segments 321 may be desirable depending upon the desired flexibility of the resulting implant.

Each adjacent pair of segments 321 may be joined by a hinge element 325 which permits lateral flexibility. In the illustrated example, the hinge element 325 comprises an axially extending strip or spine, which provides column strength along a first side of the guide tube 320. The guide tube 320 may therefore be curved by compressing a second side of the guide tube 320 which is generally offset from the spine 325 by about 180°. A limit on the amount of curvature may be set by adjusting the axial length of the space 323 between adjacent segments 321. In an example having axial spines 325, each axial spine 325 may be rotationally offset from the next adjacent axial spine 325 to enable flexibility of the overall guide tube 320 throughout a 360° angular range of motion.

Alternatively, the flexible hinge point between each adjacent segment 321 may be provided by cutting a spiral groove or plurality of parallel grooves in a tubular element in between what will then become each adjacent pair of segments 321. In this manner, each tubular element 321 will be separated by an integral spring like structure, which can permit flexibility. As a further alternative, the entire length of the guide tube 320 may comprise a spring. Each of the forgoing examples may be readily constructed by laser cutting or other cutting from a piece of tube stock, to produce a one piece guide tube 320. Alternatively, the guide tube 320 may be assembled from separate components and fabricated together using any of a variety of bonding techniques which are appropriate for the construction material selected for the tube 320.

Various distal end 314 constructions may be utilized, as will be apparent to those of skill in the art in view of the disclosure herein. In the illustrated example, the distal implant plug 316 extends within the implant 304 and is attached to the distal end of the guide tube 320. The implant plug 316 may be secured to the guide tube 320 and implant 304 in any of a variety of ways, depending upon the various construction materials. For example, any of a variety of metal bonding techniques such as a welding, brazing, interference fit such as threaded fit or snap fit, may be utilized. Alternatively, any of a variety of bonding techniques for dissimilar materials may be utilized, such as adhesives, and various molding techniques. In one construction, the implant plug 316 comprises a molded polyethylene cap, and is held in place utilizing a distal cross pin 318 which extends through the implant 304, the guide tube 320 and the implant plug 316 to provide a secure fit against axial displacement.

The proximal end 324 of the implant 304 is provided with a releasable lock 326 for attachment to a release element such as pull wire 328. Pull wire 328 extends proximally throughout the length of the tubular body 306 to a proximal pull wire control 330 on the handle 309.

As used herein, the term pull wire is intended to include any of a wide variety of structures which are capable of transmitting axial tension or compression such as a pushing or pulling force with or without rotation from the proximal end 308 to the distal end 310 of the catheter 302. Thus, monofilament or multifilament metal or polymeric rods or wires, woven or braided structures may be utilized. Alternatively, tubular elements such as a concentric tube positioned within the outer tubular body 306 may also be used as will be apparent to those of skill in the art.

In the illustrated example, the pull wire 328 is releasably connected to the proximal end 324 of the implant 304. This permits proximal advancement of the proximal end of the implant 304, which cooperates with a distal retention force provided by the core 312 against the distal end of the implant to axially elongate the implant 304 thereby reducing it from its implanted configuration to its reduced profile for implantation. The proximal end of the pull wire 328 may be connected to any of a variety of pull wire controls 330, including rotational knobs, levers and slider switches, depending upon the design preference.

In one example, the proximal end 324 of the implant 304 is provided with a releasable lock 326 for attachment of the pullwire 328 to the deployment catheter 302. In the illustrated embodiment, the releasable lock 326 is formed by advancing the pullwire 328 distally around a cross pin 329, and providing an eye or loop which extends around the core 312. As long as the core 312 is in position within the implant 304, proximal retraction of the pullwire 328 will advance the proximal end 324 of the implant 304 in a proximal direction, as shown in **FIG. 17A****.** However, following deployment, proximal retraction of the core 312 such as by manipulation of the core control 332 will pull the distal end of the core 312 through the loop on the distal end of the pullwire 328, as shown in **FIG. 17B****.** The pullwire 328 may then be freely proximally removed from the implant 304, thereby enabling detachment of the implant 304 from the deployment system 300 within a treatment site.

The implant deployment system 300 thus permits the implant 304 to be maintained in a low crossing profile configuration to enable transluminal navigation to a deployment site. Following positioning at or about the desired deployment site, proximal retraction of the core 312 enables the implant 304 to radially enlarge under its own bias to fit the surrounding tissue structure. Alternatively, the implant can be enlarged under positive force, such as by inflation of a balloon or by a mechanical mechanism as is discussed elsewhere herein. Once the clinician is satisfied with the position of the implant 304, such as by injection of dye and visualization using conventional techniques, the core 312 is proximally retracted thereby releasing the lock 326 and enabling detachment of the implant 304 from the deployment system 300.

If, however, visualization reveals that the implant 304 is not at the location desired by the clinician, proximal retraction of the pull wire 328 with respect to the core 312 will radially reduce the diameter of the implant 304, thereby enabling repositioning of the implant 304 at the desired site. Thus, the example permits the implant 304 to be enlarged or reduced by the clinician to permit repositioning and/or removal of the implant 304 as may be desired.

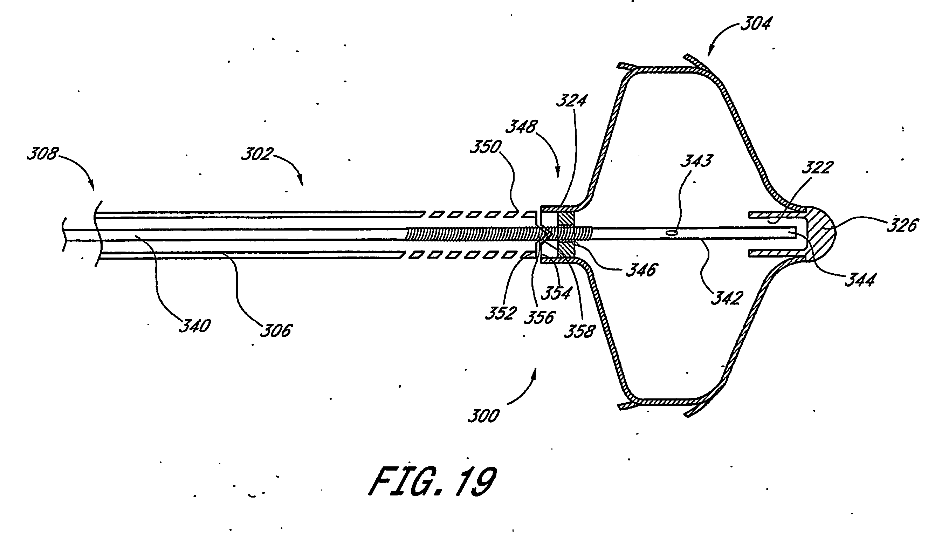

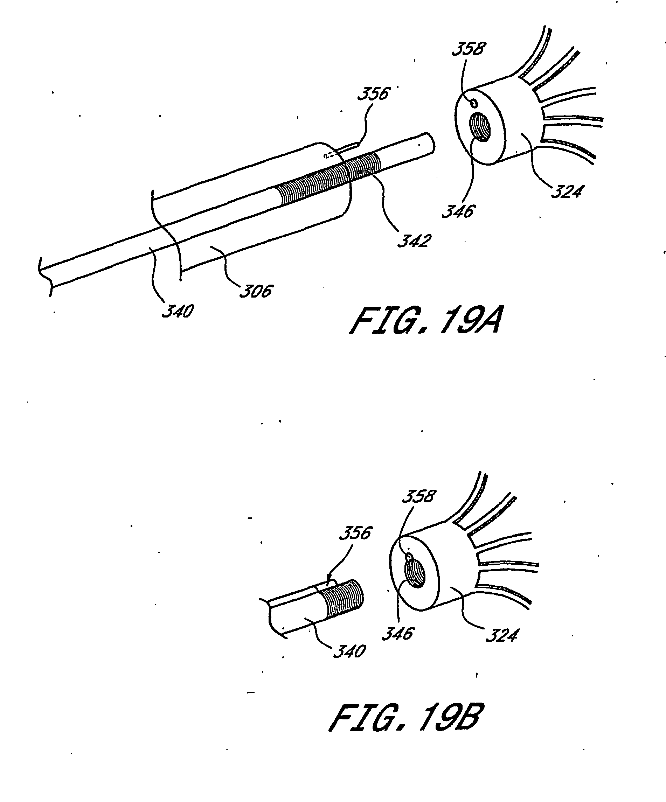

In an alternate construction, the implant may be radially enlarged or reduced by rotating a torque element extending throughout the deployment catheter. Referring to FIG. 19, the elongate flexible tubular body 306 of the deployment catheter 302 includes a rotatable torque rod 340 extending axially therethrough. The proximal end of the torque rod 340 may be connected at a proximal manifold to a manual rotation device such as a hand crank, thumb wheel, rotatable knob or the like. Alternatively, the torque rod 340 may be connected to a power driven source of rotational energy such as a motor drive or air turbine.

The distal end of the torque rod 340 is integral with or is connected to a rotatable core 342 which extends axially through the implant 304. A distal end 344 of the rotatable core 342 is positioned within a cavity 322 as has been discussed.

The terms torque rod or torque element are intended to include any of a wide variety of structures which are capable of transmitting a rotational torque throughout the length of a catheter body. For example, solid core elements such as stainless steel, nitinol or other nickel titanium alloys, or polymeric materials may be utilized. In an example intended for implantation over a guide-wire, the torque rod 340 is preferably provided with an axially extending central guidewire lumen. This may be accomplished by constructing the torque rod 340 from a section of hypodermic needle tubing, having an inside diameter of from about 0.003 cm (0.001 inches) to about 0.01 cm (0.005 inches) or more greater than the outside diameter of the intended guidewire. Tubular torque rods 340 may also be fabricated or constructed utilizing any of a wide variety of polymeric constructions which include woven or braided reinforcing layers in the wall. Torque transmitting tubes and their methods of construction are well understood in the intracranial access and rotational atherectomy catheter arts, among others, and are not described in greater detail herein. Use of a tubular torque rod 340 also provides a convenient infusion lumen for injection of contrast media within the implant 304, such as through a port 343.

The proximal end 324 of the implant 304 is provided with a threaded aperture 346 through which the core 342 is threadably engaged. As will be appreciated by those of skill in the art in view of the disclosure herein, rotation of the threaded core 342 in a first direction relative to the proximal end 324 of the implant 304 will cause the rotatable core 342 to advance distally. This distal advancement will result in an axial elongation and radial reduction of the implantable device 304. Rotation of the rotatable core 342 in a reverse direction will cause a proximal retraction of the rotatable core 342, thus enabling a radial enlargement and axial shortening of the implantable device 304.

The deployment catheter 302 is further provided with an antirotation lock 348 between a distal end 350 of the tubular body 306 and the proximal end 324 of the implant 304. In general, the rotational lock 348 may be conveniently provided by cooperation between a first surface 352 on the distal end 350 of the deployment catheter 302, which engages a second surface 354 on the proximal end 324 of the implantable device 304, to rotationally link the deployment catheter 302 and the implantable device 304. Any of a variety of complementary surface structures may be provided, such as an axial extension on one of the first and second surfaces for coupling with a corresponding recess on the other of the first and second surfaces. Such extensions and recesses may be positioned laterally offset from the axis of the catheter. Alternatively, they may be provided on the longitudinal axis with any of a variety of axially releasable anti-rotational couplings having at least one flat such as a hexagonal or other multifaceted cross sectional configuration.

As schematically illustrated in FIG. 19, one or more projections 356 on the first surface 352 may engage a corresponding recess 358 on the second surface 354. Any of a variety of alternative complementary surface structures may also be provided, as will be apparent to those of skill in the art in view of the disclosure herein. For example, referring to FIG. 19A, the projection 356 is in the form of an axially extending pin for engaging a complimentary recess 358 on the proximal end 324 of the implant 304. FIG. 19B illustrates an axially extending spline 356 for receipt within a complimentary axially extending recess 358. The various pin, spline and other structures may be reversed between the distal end of tubular body 306 and the proximal end 324 of the implant 304 as will be apparent to those of skill in the art in view of the disclosure herein.

Upon placement of the implantable device 304 at the desired implantation site, the torque rod 340 is rotated in a direction that produces an axial proximal retraction. This allows radial enlargement of the radially outwardly biased implantable device 304 at the implantation site. Continued rotation of the torque rod 340 will cause the threaded core 342 to exit proximally through the threaded aperture 346. At that point, the deployment catheter 302 may be proximally retracted from the patient, leaving the implanted device 304 in place.

By modification of the decoupling mechanism to allow the core 342 to be decoupled from the torque rod 340, the rotatable core 342 may be left within the implantable device 304, as may be desired depending upon the intended deployment mechanism. For example, the distal end of the core 342 may be rotatably locked within the end cap 326, such as by including complimentary radially outwardly or inwardly extending flanges and grooves on the distal end of the core 342 and inside surface of the cavity 322. In this manner, proximal retraction of the core 342 by rotation thereof relative to the implantable device 304 will pull the end cap 326 in a proximal direction under positive force. This may be desirable as a supplement to or instead of a radially enlarging bias built into the implantable device 304.

In the example illustrated in FIG. 19, or any other of the deployment and/or removal catheters described herein, the distal end of the tubular body 306 may be provided with a zone or point of enhanced lateral flexibility. This may be desirable in order allow the implant to seat in the optimal orientation within the left atrial appendage, and not be restrained by a lack of flexibility in the tubular body 306. This may be accomplished in any of a variety of way, such as providing the distal most one or two or three centimeters or more of the tubular body 306 with a spring coil configuration. In this manner, the distal end of the tubular body 306 will be sufficiently flexible to allow the implant 304 to properly seat within the LAA. This distal flex zone on the tubular body 306 may be provided in any of a variety of ways, such as by cutting a spiral slot in the distal end of the tubular body 306 using laser cutting or other cutting techniques. The components within the tubular body 306 such as torque rod 340 may similarly be provided with a zone of enhanced flexibility in the distal region of the tubular body 306.

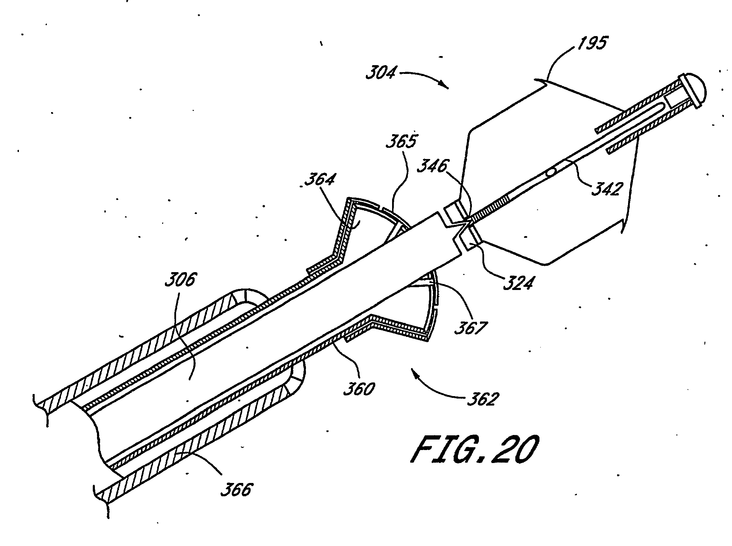

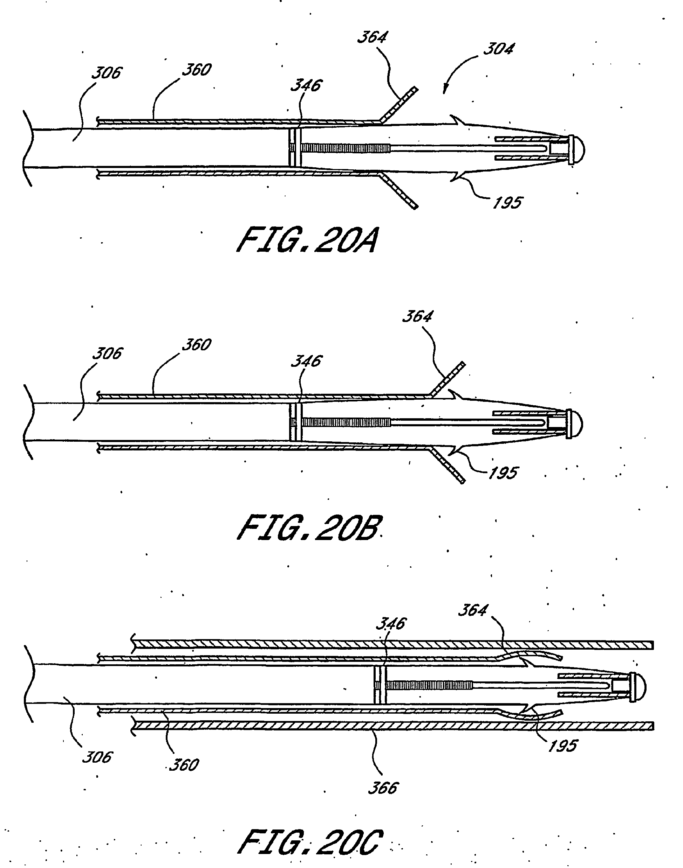

The implantable device 304 may also be retrieved and removed from the body in accordance with a further aspect of the present invention. One manner of retrieval and removal will be understood in connection with FIGS. 20 through 20C. Referring to **FIG. 20****,** a previously implanted device 304 is illustrated as releasably coupled to the distal end of the tubular body 306, as has been previously discussed. Coupling may be accomplished by aligning the tubular body 306 with the proximal end 324 of the deployed implant 304, under fluoroscopic visualization, and distally advancing a rotatable core 342 through the threaded aperture 346. Threadable engagement between the rotatable core 342 and aperture 346 may thereafter be achieved, and distal advancement of core 342 will axially elongate and radially reduce the implant 304.

The tubular body 306 is axially moveably positioned within an outer tubular delivery or retrieval catheter 360. Catheter 360 extends from a proximal end (not illustrated) to a distal end 362. The distal end 362 is preferably provided with a flared opening, such as by constructing a plurality of petals 364 for facilitating proximal retraction of the implant 304 as will become apparent. Petals 364 may be constructed in a variety of ways, such as by providing axially extending slits in the distal end 362 of the delivery catheter 360. In this manner, preferably at least about three, and generally at least about four or five or six petals or more will be provided on the distal end 362 of the delivery catheter 360. Petals 364 manufactured in this manner would reside in a first plane, transverse to the longitudinal axis of the delivery catheter 360, if each of such petals 364 were inclined at 90 degrees to the longitudinal axis of the delivery catheter 360.

In one example, a second layer of petals 365 is provided, which lies in a second, adjacent plane when the petals 365 are inclined at 90 degrees to the longitudinal axis of the delivery catheter 360. Preferably, the second plane of petals 365 is rotationally offset from the first plane of petals 364, such that the second petals 365 cover the spaces 367 fouled between each adjacent pair of petals 365. The use of two or more layers of staggered petals 364 and 365 has been found to be useful in retrieving implants 304, particularly when the implant 304 carries a plurality of tissue anchors 195.

The petals 364 and 365 may be manufactured from any of a variety of polymer materials useful in constructing medical device components such as the delivery catheter 360. This includes, for example, polyethylene, PET, PEEK, PEBAX, and others well known in the art. The second petals 365 may be constructed in any of a variety of ways. In one convenient construction, a section of tubing which concentrically fits over the delivery catheter 360 is provided with a plurality of axially extending slots in the same manner as discussed above. The tubing with a slotted distal end may be concentrically positioned on the catheter 360, and rotated such that the space between adjacent petals 365 is offset from the space between adjacent petals 364. The hub of the petals 365 may thereafter be bonded to the catheter 360, such as by heat shrinking, adhesives, or other bonding techniques known in the art.

The removal sequence will be further understood by reference to FIGS. 20A through 20C. Referring to **FIG. 20A****,** the radially reduced implant 304 is proximally retracted part way into the delivery catheter 360. This can be accomplished by proximally retracting the tubular body 306 and/or distally advancing the catheter 360. As illustrated in **FIG. 20B****,** the tubular body 306 having the implant 304 attached thereto is proximally retracted a sufficient distance to position the tissue anchors 195 within the petals 364. The entire assembly of the tubular body 306, within the delivery catheter 360 may then be proximally retracted within a transeptal sheath 366 or other tubular body as illustrated in **FIG. 20C****.** The collapsed petals 364 allow this to occur while preventing engagement of the tissue anchors 195 with the distal end of the transeptal sheath 366 or body tissue. The entire assembly having the implantable device 304 contained therein may thereafter be proximally withdrawn from or repositioned within the patient.

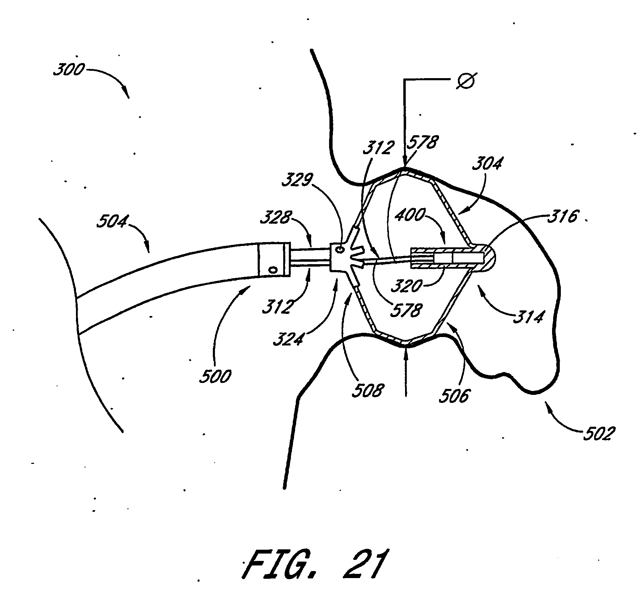

**FIG. 21** illustrates a deployment system 300, having an implant 304 and a delivery system 500, in accordance with one embodiment of the present invention. In a preferred example the implant 304 is a transluminally delivered device designed to occlude or contain particles within the left atrial appendage 502 (LAA 502) and prevent thrombus from forming in, and emboli from originating from, the LAA 502. The deployment system as described herein incorporates a slider assembly 400 such as described in U.S. Application No. 10/642,384, filed August 15, 2003.

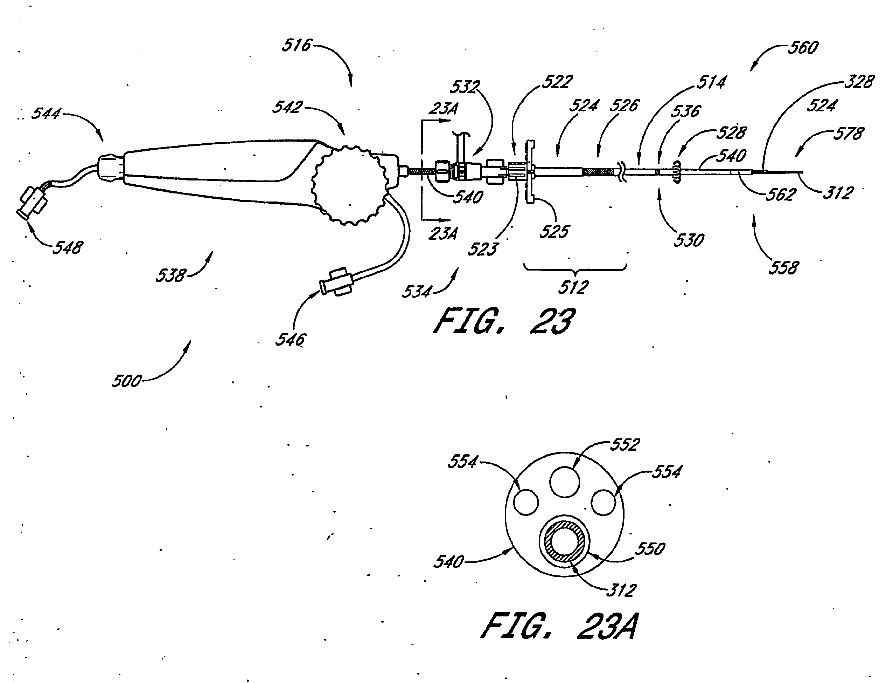

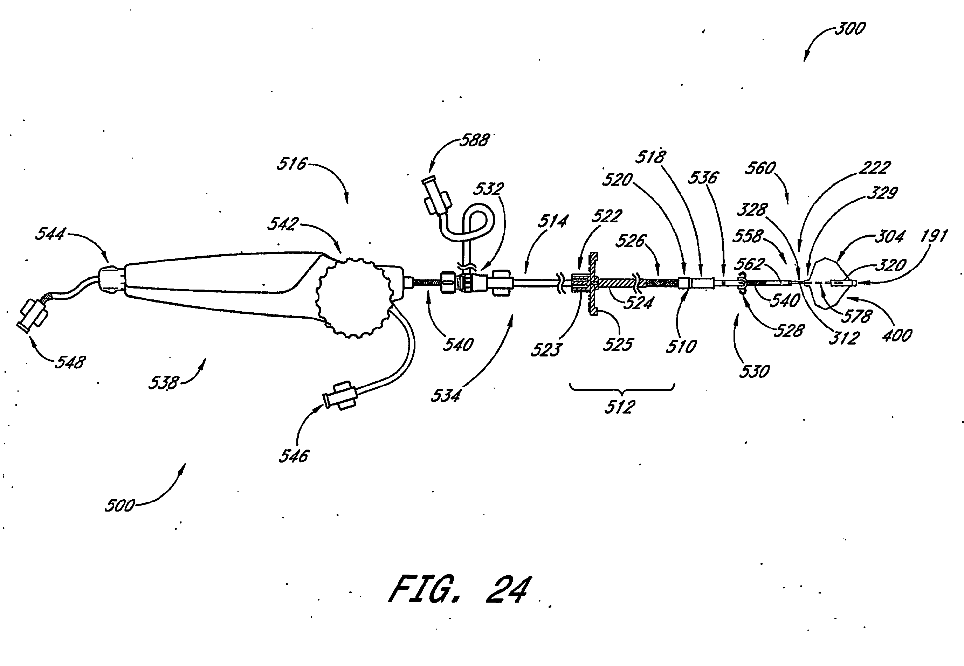

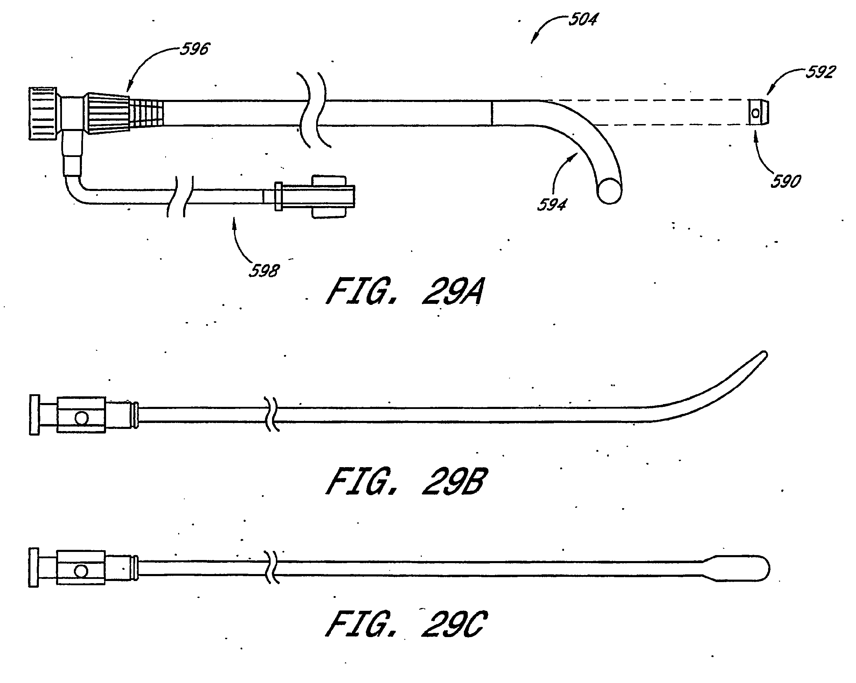

The delivery system 500 preferably may be used to deliver the implant 304 to occlude or block the LAA 502 in a patient with atrial fibrillation. The delivery system 500 preferably is compatible for use with a transseptal sheath 504, shown in FIGS. 29A-38C. The delivery system 500 and implant 304 preferably are designed to allow the implant 304 to be positioned, repositioned, and retrieved from the LAA 502 if necessary. Injection ports 546, 548, as shown in **FIG. 23** **and** **24****,** preferably are provided in the delivery system 500 to allow contrast injection proximally and distally of the implant 304 to facilitate in-vivo assessment of the positioning and seal quality of the implant 304.

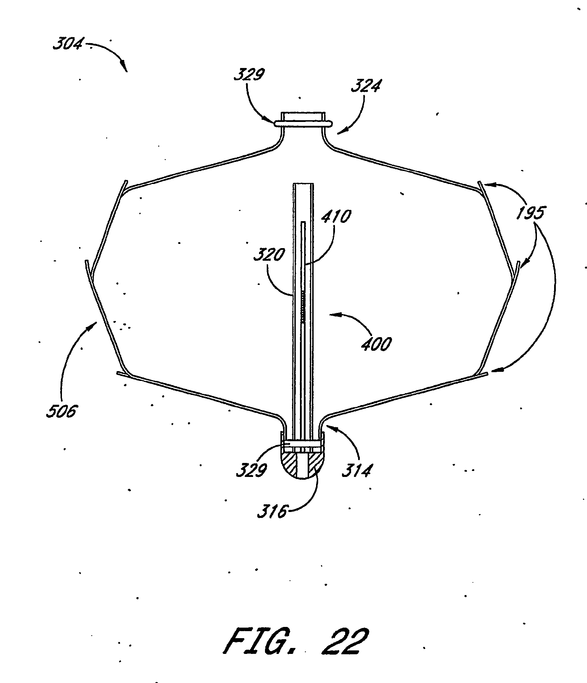

As shown in **FIG. 22****,** the implant 304 preferably is available in a range of sizes to accommodate the anatomy of a patient's LAA 502. The implant 304 preferably comprises a frame 506 and a membrane (not shown) on a proximal face of the implant, such as described above. The frame 506 preferably is constructed of self-expanding nitinol supports. The membrane preferably is constructed of a fabric covering, such as one made of ePTFE, or an ePTFE/PE laminate, such as described above. To attach the membrane to the frame 506, a PE mesh preferably is placed against the supports, with one sheet of ePTFE preferably placed over the PE mesh and another sheet of ePTFE preferably placed on an opposite side of the supports. The membrane preferably is heated on both sides causing the PE to melt into both sheets of ePTFE, thereby surrounding a portion of the frame 506. The nitinol supports allow the implant 304 to self-expand in the appendage 502, covering the orifice with the laminated fabric. The porous ePTFE/PE lamination facilitates rapid endothelialization and healing.

In one example, the membrane attached to the frame 506 is similar or identical to the barrier 15, and composite membrane 15 described above with respect to FIGS. 1-6, 10-11, and 13-16. The membrane (not shown on FIG. 22) generally has a thickness within the range of from about 0.003 cm (0.001 inches) to about 0.025 cm (0.010 inches), often less than about 0.01 cm (0.005 inches), preferably less than about 0.01 cm (0.004 inches) and in one example about 0.08 cm(0.003 inches).
The membrane generally has an average pore diameter within the range of from about 1 µm to about 100 µm, often within the range of from about 5µm to about 60µm, and in some applications, equivalent to ePTFE having an internodal distance within the range of from about 30 µm to about 60 µm.

In one example, the membrane generally has an average spacing between the edges of adjacent pores within the range of from about 50 µm to about 2,500 µm, often within the range of from about 50 µm to about 1000 µm, preferably within the range of from about 50 µm to about 300 µm, an in one example within the range of from about 75 µm to about 150 µm. The membrane generally has an open surface area of about 10% to about 50%, often about 10% to about 40%, preferably about 10% to 30% and in one example about 35%.

As shown in FIGS. 21 and 22, the implant 304 preferably extends from a proximal end or hub 324 to a distal end or hub 314. In some examples, the proximal hub 324 is coupled with a crosspin 329 as described above. In some examples the distal hub 314 is coupled with a slider assembly 400 as described above. The distal hub 314 preferably is coupled with an implant plug 316. In one example, the implant plug 316 comprises an atraumatic tip, such that contact between the atraumatic tip and the inside surface of the LAA 502 does not cause significant damage to the LAA 502. The implant 304 preferably is expandable and collapsible. The implant 304 preferably comprises anchors 195 that extend from the frame 506 when the implant 304 is expanded as described above.

As shown in **FIGS. 23** **and** **24****,** the delivery system 500 preferably comprises a loading collar 510, a peel-away sheath 512, a recapture sheath 514, a deployment catheter 516, and an axially moveable core 312, each described further below. In addition, FIG. 23 illustrates the deployment system without the a loading collar 510, and FIG. 24 illustrates the deployment system with the a loading collar 510, with the system operably connected to an implant 304.

The deployment catheter 516, which is analogous to deployment catheter 302 described above, preferably comprises a deployment handle 538 and a multi-lumen shaft 540. As shown in FIGS. 23 and 24, the deployment handle 538 preferably comprises a control knob 542, a release knob 544, a proximal injection port 546 and a distal injection port 548. The multi-lumen shaft 540 preferably comprises a four-lumen shaft shown in FIG. 23A. The multi-lumen shaft 540 preferably comprises a core lumen 550 for holding an axially moveable core 312, a control line lumen 552 and two proximal injection lumens 554 in communication with proximal injection port 546.

An axially moveable core 312 preferably extends from the deployment handle 538 through the core lumen 550 of the catheter 516 and couples the implant 304 to the delivery system 500 through a slider assembly 400 as described above. Referring to **FIGS. 21****,** **24****, and** **27****,** a control line 328 (referred to previously as a pull wire 328) preferably extends through the control line lumen 552 and preferably couples a proximal hub 324 of the implant 304 to the deployment handle control knob 542, allowing for implant 304 expansion and collapse. The control line 328 preferably extends around a portion of the axially movable core 312 near the proximal hub 324 of the implant 304, and is coupled to the implant 304 by crosspin 329, as described above.

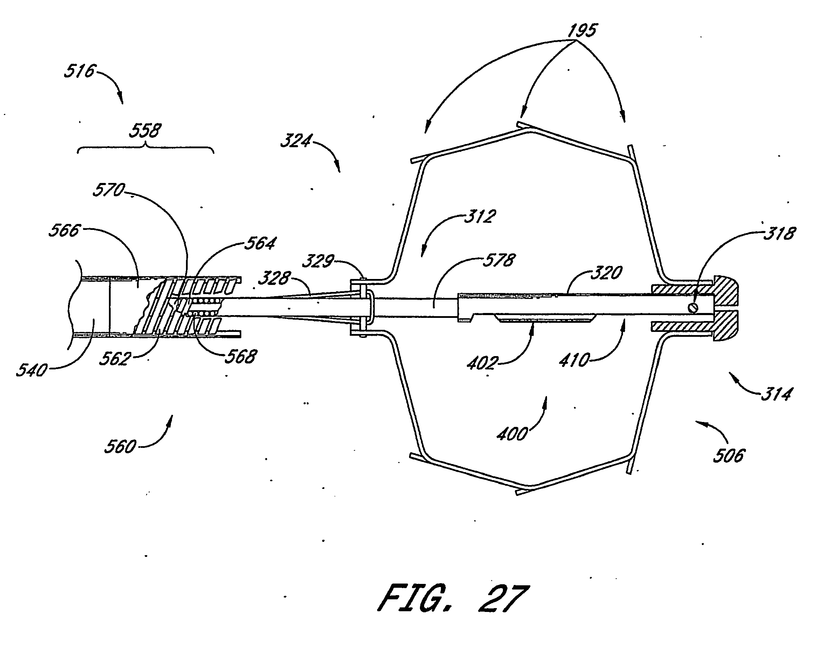

As shown in **FIG. 27****,** the deployment catheter 516 preferably comprises a flexible catheter section 562 at its distal end, which in some examples is a spiral cut tubular section housed in a polymer sleeve 566. The flexible catheter section 562 may be coupled to a distal end of multilumen shaft 540.

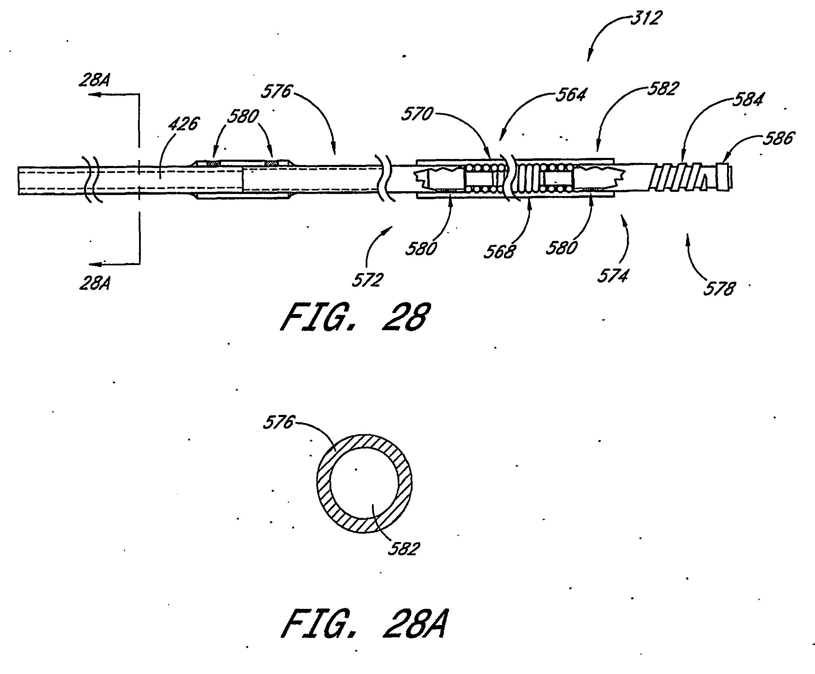

As shown in **FIGS. 27** **and** **28****,** the axially moveable core 312 preferably includes a hollow proximal shaft 576 and a hollow distal shaft 578 with a flexible hollow core section 564 therebetween, all co-axially aligned and connected. In one example, the proximal end of the distal shaft 578 is attached to the distal end of the flexible core section 564, and the proximal end of the flexible core section 564 is attached to the distal end of the proximal shaft 576. In some examples, the flexible core section 564 has a spring coil section 568 housed in a polymer sleeve 570, the spring coil section 568 preferably coupled with the shafts 576 and 578 on first and second ends 572, 574. ,

The axially moveable core 312 preferably is disposed within the deployment catheter 516 such that the flexible core section 564 may be linearly co-located with the flexible catheter section 562 at a distal portion 560 of the delivery system 500 during appropriate times during a procedure, as shown in FIG. 27. When the flexible core section 564 is aligned and linearly co-located with the flexible catheter section 562, the sections preferably cooperate to form a delivery system flexible segment 558. As shown in **FIGS. 23****,** **24****, and** **27****,** the delivery system flexible segment 558 preferably is located toward a distal end 560 of the delivery system 500.

In one example, shown in FIG. 28, the distal shaft 578m flexible core section 564, and proximal shaft 576 are attached by welding. Small windows 580 may be provided to allow welding materials to flow between the shafts 564, 576 and 578 and provide stronger bonding therebetwee. In another example, solder, glue, or press-fitting is used to attach shafts 564, 576, and 578 to one another, as is well known to those of skill in the art. In another example, the shafts 564m 576 and 578 are formed from a single tube, for example, laser-cut tube. In other examples, more than one tube may be used to form each of the shafts 564, 576 and 578. For example, FIG. 28 illustrates proximal shaft 576 comprising two tubes connected by welding such as described above.

Referring to **FIG. 28A****,** distal contrast media preferably can be injected through a lumen 582 in the shafts 576 and 578 for determining the placement of the implant 304. This lumen may be in fluid communication with distal injection port 548, shown in FIGS. 23 and 24. The distal shaft 578 preferably comprises a mating surface 584 and a radiopaque marker 586, such as described above. In one example, the mating surface 584 is a threaded surface. The distal shaft 578 preferably is releasably coupled through the implant 304 with the slider assembly 400, such as described above.

When the delivery system 500 is assembled, the recapture sheath 514 is preferably loaded over the deployment catheter 516, distal to the handle 538, as shown in FIGS. 23 and 24. The recapture sheath 514 preferably is designed to allow recapture of the implant 304 prior to its final release such as described with respect to retrieval catheter 360 above. Recapture petals or flares 528 preferably are provided on the distal end 530 of the recapture sheath 514 to cover the anchors 195 of the implant 304 during retrieval into the transseptal sheath 504, as described below. A Touhy-Borst adapter or valve 532 preferably is attached to the proximal end 534 of the recapture sheath 514. The recapture sheath 514 preferably comprises a radiopaque marker 536 on its distal end 530 near the recapture flares 528. The recapture sheath 514 preferably comprises a recapture sheath injection port 588 for delivering fluid proximal the implant 304.

The peel-away sheath 512 preferably is provided over a portion of the recapture sheath 514, between Touhy-Borst valve 532 and recapture flares 528. The peel-away sheath 512 preferably is used to introduce the delivery system 500 into a transseptal sheath 504 shown in **FIGS. 29A-C**, described below. As shown in FIGS. 23 and 24, the peel-away sheath 512 preferably comprises a locking collar 522, a peel-away section 524, and a reinforced section 526. The locking collar can be unlocked relative to peel-away section 524, and preferably includes a threaded hub 523 that releasably engages tabs 525 of the peel-away section 524.

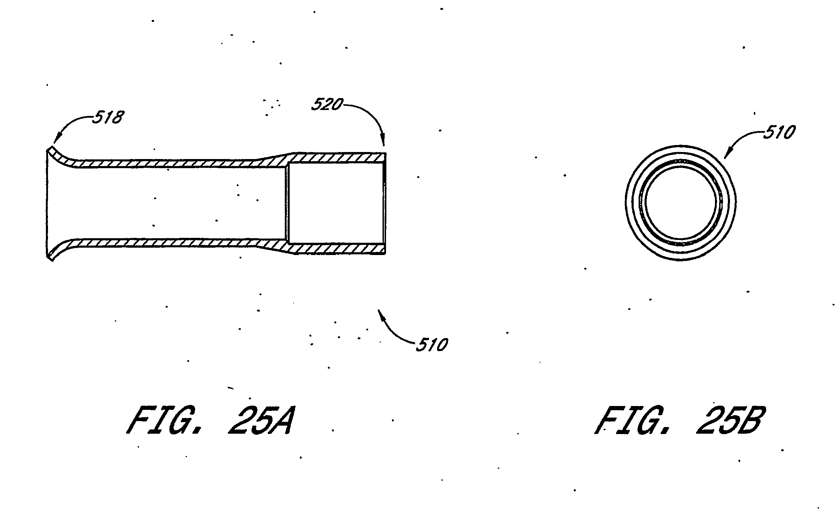

The loading collar 510 preferably is located over a portion of the peel-away sheath 512 and a portion of the recapture sheath 514 with its proximal end being located over the peel-away sheath 512 at its distal end loaded over recapture sheath 514. The loading collar 510 preferably accommodates loading a collapsed implant 304 into the peel-away sheath 512 as described below. As shown in **FIGS. 24** **and** **25****,** the loading collar 510 preferably comprises a first end portion 518 adapted to receive and extend over a collapsed implant 304, and a second end portion 520 configured to guide the collapsed implant 304 into the peel-away sheath 512. The loading collar 510 preferably is made of stainless steel.

To assemble the delivery system, the axially movable core 312 and control line 328 preferably are fed into the multi-lumen shaft 540 of the deployment catheter 516. The multi-lumen shaft 540 preferably is then coupled with components of the deployment handle 538 and the injection port components 546, 548. The recapture sheath 514 preferably is then slid onto the deployment catheter 516. The peel-away sheath 512 and the loading collar 510 preferably are then slid onto the recapture sheath 514, and the recapture sheath 514 is slid onto the deployment catheter 516. The implant 304 preferably is then loaded on an end of the axially movable core 312 and coupled with the control line 328. In one embodiment, the implant 304 is loaded on an end of the axially movable core 312 by screwing the axially movable core 312 into the slider nut 402 of the slider assembly 400. The control knob 542 and outer casing of the deployment handle 538 preferably are then coupled with the system.

The deployment system 300 preferably is used in connection with a transseptal sheath 504 to advance the implant 304 for deployment in a patient. As shown in **FIGS. 21** **and** **29A-C**, the transseptal sheath 504 is a tubular device that in one example can be advanced over a guidewire (not shown) for accessing the LAA 502 of a patient. Transseptal sheath 504 in one embodiment has a permanent bend 594, as shown in the views of FIGS. 29A and 29B. A hemostasis valve 596 is provided at the proximal end of transseptal sheath. A fluid injection port 598 is also provided at the proximal end to delivery fluid such as contrast media through the transseptal sheath. Systems and methods for implanting the device 304 in the LAA 502 are described further below.

In one example, the system and method preferably allows for access and assessment of the LAA 502. A guidewire (not shown) preferably is used to access the superior vena cava through groin access. A transseptal sheath 504 preferably is advanced over the guidewire and into the superior vena cava. The guidewire preferably is removed and replaced with a transseptal needle (not shown). The transseptal sheath 504 preferably is retracted inferiorly so that the bend 594 in transseptal sheath directs the distal tip of the transseptal sheath toward the fossa ovalis. The needle preferably is advanced to puncture the fossa ovalis. The transseptal sheath 504 preferably is advanced to establish access to the LAA 502 and the needle preferably is retracted. Further details or disclosure are provided in copending U.S. Patent Applications Serial Nos. 09/435,562 and 10/033,371.

After properly preparing a transseptal sheath 504 for LAA 502 access, the size of the neck diameter and morphology of the LAA 502 preferably is determined by advancing the transseptal sheath 504 to the distal portion of the LAA 502 and injecting contrast media to obtain an initial left atrial appendogram. The neck diameter preferably is measured approximately 5 mm in from the ostium of the LAA 502 at end diastole.

In one example, the system and method preferably allows for selection and preparation of a deployment system 300. A deployment system 300 preferably comprises an implant 304 of an appropriate size for placement in a patient. Initially, the implant 304 preferably is in an expanded configuration, with axially moveable core 312 engaging slider assembly 400, as described above. The recapture sheath 514 preferably is positioned so it covers and supports the flexible segment 558 of the delivery system 500, wherein the flexible catheter section 562 of deployment catheter 302 and flexible core section 564 of axially moveable core 312 are aligned. The Touhy-Borst valve 532 preferably is tightened over the deployment catheter 516 to prevent relative movement between recapture sheath 514 and deployment catheter 516. The loading collar 510 and peel-away sheath 512 preferably are positioned so they are at the base of the recapture flares 528, proximal thereto.

The delivery system 500 preferably is loaded by rotating the control knob 542 counterclockwise until the implant 304 is fully collapsed. Preferably, at least a portion of the control line 328 is coupled with the control knob 542 such that rotation of the control knob 542 in the counterclockwise direction retracts at least a portion of the control line 328. Retraction of the control line 328 preferably places tension on the proximal hub 324 of the implant 304, because a portion of the control line 328 preferably is coupled with the proximal hub 324 by a pin 329. While the distal portion of the axially moveable core 312 engages slider assembly 400 and applies a distal force to distal hub 314 of the implant 304, tension in the control line 328 preferably causes the proximal hub 324 of the implant 304 to move proximally relative the axially moveable core 312, thereby collapsing the implant 304.

The diameter of the implant 304 preferably is reduced to approximately 1/3^{rd} or less of its original diameter when collapsed. The loading collar 510 and peel-away sheath 512 are then advanced distally over the flares 528 and implant 304 until the distal tip of the implant 304 is aligned with the distal end of the peel-away sheath 512 and the distal end of the loading collar is about 1.5 cm from the distal tip of the implant At this point, the flares 528 partially cover the implant. The loading collar 510 preferably is removed and discarded.

With the implant 304 partially within the recapture sheath 514 and retracted within the peel-away sheath 512, the entire system preferably is flushed with sterile heparinized saline after attaching stopcocks to the recapture sheath injection port 588, the proximal injection port 546 and distal injection port 548 of the delivery system 500. The recapture sheath 514 and the Touhy-Borst valve 532 are first thoroughly flushed through port 588. Then the distal injection port 548 and the proximal injection port 546 of the deployment handle 538 are preferably flushed through. The distal injection port 548 is in fluid communication with lumen 426 of axially moveable core 312, and proximal injection port 546 is in fluid communication with injection lumens 554 of multilumen shaft 540. The transseptal sheath 504 placement preferably is reconfirmed using fluoroscopy and contrast media injection.

The delivery system 500, as described above, with implant 304 inserted therein, preferably is then inserted into the proximal end of the transseptal sheath 504. To avoid introducing air into the transseptal sheath 504 during insertion of the delivery system 500, a continual, slow flush of sterile heparinized saline preferably is applied through the proximal injection port 546 of the deployment handle 538 to the distal end of the deployment catheter 516 until the tip of the peel-away sheath 512 has been inserted into, and stops in, the hemostatic valve of the transseptal sheath 504. Preferably, the distal tip of the peel-away sheath 512 is inserted approximately 5 mm relative to the proximal end of the transseptal sheath 504.

Under fluoroscopy, the recapture sheath 514 and deployment catheter 516 preferably are advanced, relative to the peel-away sheath 512, approximately 20-30 cm from the proximal end of the transseptal sheath, and the system 500 preferably is evaluated for trapped air. The peel-away sheath 512 is preferably not advanced into the transseptal sheath 504 due to the hemostasis valve 596 blocking its passage. If air is present in the system 500, it may be removed by aspirating through the distal injection port 548, recapture sheath injection port 588, or proximal injection port 546. If air cannot be aspirated, the deployment catheter 516 and recapture sheath 514 preferably are moved proximally and the delivery system 500 preferably is removed from the transseptal sheath 504. All air preferably is aspirated and the flushing/introduction procedure preferably is repeated.

The peel-away sheath 512 preferably is manually slid proximally to the proximal end 534 of the recapture sheath 514. The Touhy-Borst valve 532 preferably is loosened and the deployment catheter 516 preferably is advanced distally relative to the recapture sheath 514 until the deployment handle 538 is within about 2 cm of the Touhy-Borst valve 532 of the recapture sheath 514. This causes the implant 304 to be advanced distally within the transseptal sheath 504 such that the recapture sheath 514 no longer covers the implant 304 or the flexible section 558. The Touhy-Borst valve 532 preferably is tightened to secure the deployment catheter 516 to fix relative movement between the deployment catheter 516 and recapture sheath 514.

Under fluoroscopy, the implant 304 preferably is advanced to the tip of the transseptal sheath 504 by distal movement of the delivery catheter 302. The distal hub 314 of implant 304 preferably is aligned with a transseptal sheath tip radiopaque marker 590. Under fluoroscopy, the sheath 504 positioning within the LAA 502 preferably is confirmed with a distal contrast media injection.

The position of the implant 304 preferably is maintained by holding the deployment handle 538 stable. The transseptal sheath 504 preferably is withdrawn proximally until its tip radiopaque marker 590 is aligned with the distal end of the deployment catheter flexible segment 558. This preferably exposes the implant 304.

Under fluoroscopy, the implant 304 preferably is expanded by rotating the control knob 542 clockwise until it stops. Rotating the control knob 542 preferably releases tension on the control line 328, preferably allowing the implant 304 to expand. The implant 304 preferably is self-expanding. After expansion, any tension on the LAA 502 preferably is removed by carefully retracting the deployment handle 538 under fluoroscopy until the radiopaque marker 586 on the axially movable core 312 moves proximally approximately 1-2 mm in the guide tube 320. The position of the implant 304 relative the LAA 502 preferably is not altered because the axially movable core 312 preferably is coupled with the slider assembly 400 allowing for relative movement between the implant 304 and the axially movable core 312. The slider assembly 400 preferably allows for the distal portion of the axially movable core 312 to be slightly retracted proximally from the distal hub 314 of the implant 304, thereby removing any axial tension that may be acting on the implant 304 through the axially movable core 312. The radiopaque marker 586 preferably is about 1-2 mm proximal from the implant 304 distal hub 314, and the transseptal sheath 592 tip preferably is about 2-3 mm proximal from the implant proximal hub 324, thereby indicating a neutral position.

Under fluoroscopy, the expanded diameter (∅ in FIG. 21) of the implant 304 preferably is measured in at least two views to assess the position of the implant within the LAA 502. The measured implant diameter ∅ preferably is compared to the maximum expanded diameter.

Preferably, the labeled proximal and distal injection ports 546, 548 of the deployment handle 538 shown in FIG. 23, correlate with the proximal and distal contrast media injections. The proximal contrast media injections are delivered through the delivery catheter lumen 554 to a location proximal to the implant 304. The distal contrast media injections are delivered through the axially movable core 312 to a location distal to the implant 304. Proximal contrast media injections preferably are completed in two views. If the injection rate is insufficient, the recapture sheath injection port 588 may be used independently or in conjunction with the proximal injection port 546 to deliver fluid to a location proximal to the implant 304.

If satisfactory results are seen, any transverse tension on the LAA 502 preferably is released by exposing the flexible segment 558 of the delivery system 500. The flexible catheter section 562 and the flexible core section 564 preferably are linearly co-located to cooperate as the flexible segment 558 of the delivery system 500. This preferably is accomplished by retracting the transseptal sheath 504 proximally approximately 2 cm to expose the flexible segment. By exposing the flexible segment 558, the flexible segment 558 preferably will flex to allow the implant 304 to sit within the LAA 502 free from transverse forces that may be created, for example, by contractions of the heart acting against the transseptal sheath 504 or deployment catheter 516.

Once the flexible segment 558 is exposed, distal contrast media injections preferably are completed in at least two views to verify proper positioning of the implant 304. A flush of saline preferably is used as needed between injections to clear the contrast media from the LAA 502. Following the contrast media injections, the transseptal sheath 504 preferably is advanced distally to cover the flexible segment 558.

If implant 304 position or results are sub-optimal, the implant 304 preferably may be collapsed and repositioned in the LAA 502. To achieve this, under fluoroscopy, the deployment handle 538 preferably is advanced distally to place the radiopaque marker 586 of the axially moveable core 312 at the distal hub 314 of the implant 304. The distal end of the transseptal sheath 504 preferably is aligned with the distal end of the flexible segment 558. The control knob 542 preferably is rotated until the implant 304 has been collapsed to approximately 1/3^{rd} or less of its expanded diameter. The control knob 542 preferably acts on the control line 328 to place tension on the proximal hub 324 of the implant 304, pulling the proximal hub 324 of the implant 304 proximally relative the distal hub 314 of the implant 304 to collapse the implant 304. The implant 304 preferably can be repositioned and re-expanded.

The stability of the implant 304 preferably is verified in several views. Stability tests preferably are preformed in the following manner. A contrast media filled syringe preferably is connected to the distal injection port 548 of the deployment handle 538. Under fluoroscopy, at least about a 10 mm gap between the tip of the transseptal sheath 504 and the proximal hub 222 of the implant 304 is preferably confirmed.

The stability of the implant 304 in the LAA 502 preferably is evaluated using fluoroscopy and echocardiography. The recapture sheath Touhy-Borst valve 532 preferably is loosened. Then the deployment handle 538 preferably is alternately retracted and advanced about 5-10 mm while maintaining the position of the transseptal sheath 504 and simultaneously injecting contrast media through the distal injection port 548. This tests how well the implant is held within the LAA 502.

If the implant stability tests are unacceptable, the implant 304 preferably may be collapsed and repositioned as described above. If repositioning the implant 304 does not achieve an acceptable result, the implant 304 preferably may be collapsed and recaptured as described further below.

The implant 304 preferably meets the following acceptance criteria, associated with the assessment techniques listed below, prior to being released. The assessment techniques to be evaluated preferably include 1) residual compression; 2) implant location; 3) anchor engagement; 4) seal quality; and 5) stability. For residual compression, the implant diameter ∅, as measured by fluoroscopic imaging, preferably is less than the maximum expanded diameter of the implant 304. For implant location, the proximal sealing surface of the implant 304 preferably is positioned between the LAA 502 ostium and sources of thrombus formation (pectinates, secondary lobes, etc.) (preferably imaged in at least two views). For anchor engagement, the implant frame 506 preferably is positioned within the LAA 502 so as to completely engage a middle row of anchors 195 in an LAA 502 wall (preferably imaged in at least two views). For seal quality, the contrast injections preferably show leakage rated no worse than mild (preferably defined as a flow of contrast media, well defined, and filling one-third of the LAA 502 during a proximal injection over a period of up to about five ventricular beats, preferably imaged in at least two views). For stability, there preferably is no migration or movement of the implant 304 relative to the LAA 502 wall as a result of the Stability Test.

If implant recapture is necessary, because a different size implant 304 is necessary or desired, or if acceptable positioning or sealing cannot be achieved, the implant 304 preferably is fully collapsed as described above. Once the implant 304 is collapsed, the locking collar 522 of the peel away sheath 512 preferably is unlocked. The peel-away portion 524 of the peel-away sheath 512 preferably is split up to the reinforced section 526 and removed. The reinforced section 526 of the peel-away sheath 512 preferably is slid proximally to the hub of the recapture sheath 514. The Touhy-Borst valve 532 on the proximal end of the recapture sheath 514 preferably is slightly loosened to allow smooth movement of the sheath 514 over deployment catheter 516 without allowing air to enter past the Touhy-Borst valve 532 seal. By removing the peel-away portion 524 of peel-away sheath 512, the recapture sheath 514 can now be advanced further distally relative to the transseptal sheath.

While holding the deployment catheter 516 and transseptal sheath 504 in place, the recapture sheath 514 preferably is advanced distally into the transseptal sheath 504 until a half marker band 536 on the recapture sheath 514 is aligned with a full marker band 590 on the transseptal sheath 504. This preferably exposes the recapture flares 528 outside the transseptal sheath.

The collapsed implant 304 preferably is retracted into the recapture sheath 514 by simultaneously pulling the deployment handle 538 and maintaining the position of the recapture sheath 514 until approximately half the implant 304 is seated in the recapture sheath 514. The Touhy-Borst valve 532 on the recapture sheath 514 preferably is tightened over the deployment catheter 516. The recapture sheath 514 and implant 304 preferably are retracted into the transseptal sheath 504 by pulling on the recapture sheath 514 while maintaining the position of the transseptal sheath 504, preferably maintaining left atrial access. The recapture flares 528 of the recapture sheath 514 preferably cover at least some of the anchor elements 195 on the implant 304 as the implant is retracted proximally into the transseptal sheath 504.

If the implant's position and function are acceptable, and implant recovery recapture is not necessary, the implant 304 preferably is released from the delivery system 500. Under fluoroscopy, the transseptal sheath 504 preferably is advanced to the proximal hub 324 of the implant 304 for support. The release knob 544 on the proximal end of the deployment handle 538 preferably is rotated to release the implant 304. Rotating the release knob 544 preferably causes a threaded portion 584 of the distal shaft 578 of the axially movable core 312 to rotate with respect to the slider assembly 400 such that the threaded section 584 preferably is decoupled from the slider assembly 400. Under fluoroscopy, after the axially movable core 312 is decoupled from the implant 304, the release knob 544 preferably is retracted until the distal end 578 of the axially movable core 312 is at least about 2 cm within the transseptal sheath 504.

Under fluoroscopy, while assuring that transseptal access is maintained, the delivery system 500 preferably is retracted and removed through the transseptal sheath 504. Under fluoroscopy, the transseptal sheath 504 position preferably is verified to be approximately 1 cm away from the face of the implant 304. Contrast injections, fluoroscopy and/or echocardiography preferably may be used to confirm proper positioning and delivery of the implant 304 and containment of the LAA 502. The transseptal sheath 504 preferably is withdrawn.

Throughout this application the terms implant and occlusion device have been used. One of ordinary skill in the art will appreciate that all of the disclosures herein are applicable to a wide variety of structures that include both implants that may or may not also be occlusion devices. Routine experimentation will demonstrate those limited circumstances under which certain disclosures and combinations thereof are not beneficial.

Further details regarding left atrial appendages devices and related methods are disclosed in U.S. Patent No. 6,152,144, titled "Method and Device for Left Atrial Appendage Occlusion," filed November 6, 1998, U.S. Patent Application Serial No. 09/435,562, filed November 8, 1999, U.S. Patent Application Serial No. 10/033,371, titled "Method and Device for Left Atrial Appendage Occlusion," filed October 19, 2001, and U.S. Patent Application Serial No. 10/642,384, titled "System and Method for Delivering a Left Atrial Appendage Containment Device," filed August 15, 2003.

### I. COMPOSITE LAMINATION EMBODIMENTS - COMPOSITE LAMINATION STENTS

In one embodiment, a thin film composite lamination, such as described with respect to FIGS. 13-16, is provided with a stent. The stent may be self-expanding, or balloon expandable, as is well known to those of skill in the art. In one embodiment, the stent is cylindrical in shape, and may be adjusted from a first, reduced diameter configuration to a second, expanded diameter configuration. The stent is placed in the first, reduced diameter configuration for transluminal delivery to a treatment site, and then expanded to its second, expanded diameter configuration once positioned at the treatment site.

Self expanding stents are generally restrained in the first, reduced diameter configuration during delivery with a diametrical restraint. In one embodiment, the diametrical restraint is removed at the treatment site, and the stent self expands by release of stored elastic energy. Balloon expandable stents are generally delivered in the first, reduced diameter configuration without use of a diametrical restraint, and are generally diametrically expanded by a balloon catheter. Balloon expandable stents generally plastically deform to a larger diameter during expansion, and the enlarged diameter is substantially retained once the balloon catheter or other expansion device is removed.

In one embodiment, the stent is bare metal, coated, or covered, and may be self expanding or balloon expandable, as described in greater detail above. In one embodiment, a bare metal stent consists of metal struts and substantially no additional coatings, layers, or other materials applied to the strut surface.. In one embodiment, a coated stent includes a coating material applied to the struts of the stent. The coating material may be biological in nature, such as, for example, heparin, or the coating material may be a synthetic polymer, such as, for example, silicone or polyurethane. In one embodiment, the coating material is applied by dipping or spraying the coating material onto the stent, although other methods may be employed, as are well known to those of skill in the art. In one embodiment, the coating is comprised of a drug intended to interact with a vessel lumen, for example rapamycin intended to reduce luminal restenosis.

In one embodiment, the stent is a covered stent, which includes a stent covered by a covering material. In one embodiment, the covering material includes Dacron, ePTFE, or silicone. The covering material may be applied as a sheet so as to substantially cover or occlude pores in the walls of the stent. The pores in the wall of the stent may be formed by the crossing or proximity of stent struts to one another. The covering material may be applied by bonding, attaching, or laminating sheets of covering material to the stent.

In one embodiment, drugs are applied to the stent, the coating material, or the covering material. The drugs may be engineered such that they leach out from the stent, coating material, or covering material over time. In one embodiment, a drug coated stent comprises a stent in which a drug has been incorporated into the coating material.

In one embodiment, the stent includes a coil stent or a cut tube stent. A coil stent is generally a self expanding single strand of wire wrapped into a spring coil-like shape, and bare metal. In one embodiment, a cut tube stent is self expanding or balloon expandable, bare metal, coated, and covered. In one embodiment, a cut tube stent is cut from metal tubes with a laser.

In one embodiment, a covered stent provides a barrier between a vessel wall and a vessel lumen. In one embodiment, a covered stent is deployed in a degenerated sapphenous vein graft to prevent grumous material from embolizing. Degenerated sapphenous vein grafts are often used to bypass coronary artery blockages, and are generally lined with friable grumous material that can easily dislodge and float downstream as emboli and cause associated tissue ischemia. In addition, arterial vessels in the body may contain unstable atheromatous plaques. When plaques rupture, thrombogenic surfaces may be created, and clots may for at, and embolize from, the ruptured plaque site. In one embodiment, a covered stent is used to prevent such consequences following plaque rupture. In venous vessels and in some arterial conduits, such as the superficial femoral artery or neurological arteries, thrombus may be found. The thrombus may be dislodged from the site, or may reform at the site after removal due to vessel wall characteristics. In one embodiment, a covered stent is used to trap thrombus between the stent and the vessel wall. In another embodiment, the covered stent also provides smooth lumen, or a less thrombogenic surface for blood flow. In another embodiment, a covered stent is used to seal a vessel lumen if the vessel wall ruptures or is punctured during medical interventions. In yet another embodiment, a covered stent is used as a conduit between two vessels for the purpose of bypass or flow redirection. In another embodiment, a covered stent is used as a conduit between a vessel and a chamber of the heart for the the purpose of bypass or flow redirection.

It has been found that it may be undesirable to place an impermeable membrane between a vessel lumen and a vessel wall. It may be important to allow communication of biochemical species between the vessel lumen and the vessel wall. In one embodiment, a covered stent comprises a porous covering material.

In one embodiment, a thin, flexible, ePTFE layer is applied to a cut tube stent using the bonding and/or lamination techniques described above to create a composite lamination. In one embodiment, the composite lamination is compressed into a low profile delivery configuration, with a reduced diameter, for transluminal delivery to a treatment site. In another embodiment, the composite lamination does not inhibit expansion of a self expanding or balloon expandable stent. In another embodiment, the composite lamination comprises a porosity sufficient to avoid excluding communication between a vessel wall and a vessel lumen into which the composite lamination is inserted. In another embodiment, the composite lamination provides superior and/or improved biocompatibility through the selection of ePTFE as the covering material, and polyethylene as the bonding layer.

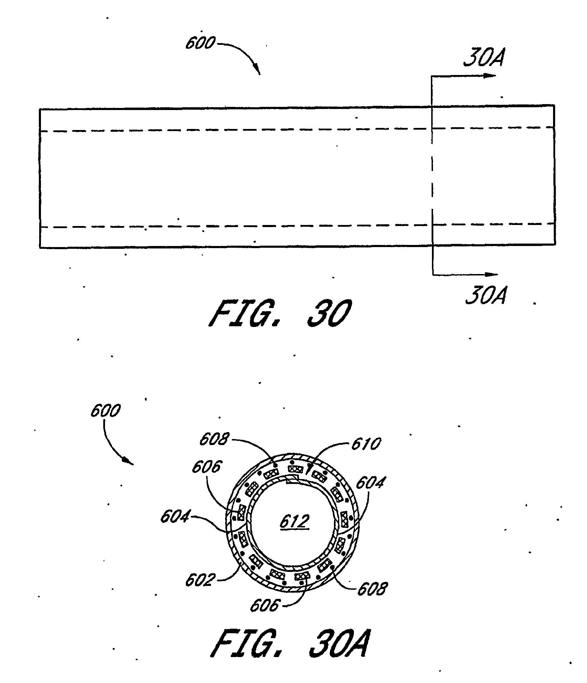

One embodiment of a composite lamination stent is illustrated in **FIG. 30** and **FIG. 30A****.** Composite lamination stent 600 comprises outer layer 602, inner layer 604, bonding layer 606, and stent 608. In one embodiment, outer layer 602 and inner layer 604 comprise sheets of expanded polytetrafluoroethylene (ePTFE) wrapped circumferentially about the stent 608. In one embodiment, the edges of inner layer 604 overlap to form overlapping region 610.

In one embodiment, the outer layer 602 and inner layer 604 are similar or identical to the first and second membranes 250, 252 described in greater detail above. The bonding layer 606 may be an open mesh bonding layer similar or identical to bonding layer 254 described above. In one embodiment, the bonding layer 606 has an open surface area within the range of from about 20% to about 70%, often from about 30% to about 60%, and in some applications, from about 40% to about 50%, depending on the desired clinical performance.

The average pore diameter of the bonding layer 606 is preferably within the range of from about 0.01 cm (0.005 inches) to about 0.13 cm (0.050 inches), often from about 0.051 cm (0.020 inches) to about 0.10 cm (0.040 inches), and in some applications from about 0.038 cm (0.015 inches) to about 0.076 cm (0.030 inches), depending on the desired clinical performance, and in one embodiment, is about 0.051 cm (0.020 inches).

The thickness of the bonding layer 606 can be varied widely, and is generally within the range of from about 0.001 cm (0.0005 inches) to about 0.01 cm (0.005 inches), often from about 0.002 cm (0.0008 inches) to about 0.01 cm (0.004 inches), and in some applications from about 0.002 cm (0.0009 inches) to about 0.008 cm (0.003 inches) depending on the desired clinical performance. In one embodiment, the bonding layer 606 has a thickness of about 0.003 cm (0.001 inches) to about 0.005 cm (0.002 inches). The optimum thickness can be determined for any particular application and for any particular material or combination of materials through routine experimentation by one of ordinary skill in the art based on the disclosure herein.

The outer and inner layers 602, 604 may have either the same or different thicknesses, and generally have a thickness within the range of from about 0.001 cm (0.0005 inches) to about 0.025 cm (0.010 inches), preferably from about 0.002 cm (0.0007 inches) to about 0.02 cm (0.008 inches), often from about 0.002 cm (0.0008 inches) to about 0.01 cm (0.005 inches), and in certain applications, from about 0.002 cm (0.0009 inches) to about 0.008 cm (0.003 inches). In one embodiment, the outer and inner layers 602, 604 each have a thickness on the order of from about 0.003 cm (0.001 inches) to about 0.005 cm (0.002 inches). The optimum thickness can be determined for any particular application and for any particular material or combination of materials through routine experimentation by one or ordinary skill in the art based on the disclosures herein.

The outer and inner layers 602, 604 may have either the same or different average pore diameters. The average pore diameter is often within the range of from about 1 µm to about 200 µm, preferably within the range of from about 5 µm to about 100 µm, and in some applications within the range of from about 10 µm to about 70 µm. The optimum average pore diameter or spacing can be determined for any particular application and for any particular material or combination of materials through routine experimentation by one or ordinary skill in the art based on the disclosures herein.

The outer and inner layers 602, 604 may have either the same or different average spacing between the edges of adjacent pores, and generally have an average internodal distance of from about 10 µm to about 100 µm, or exhibit equivalent cellular migration or ingrowth characteristics as layers having such internodular distances.

The resulting lamination of the composite lamination stent 600 generally has a thickness within the range of from about 0.003 cm (0.001 inches) to about 0.025 cm (0.010 inches), often less than about 0.01 cm (0.005 inches), preferably less than about 0.01 cm (0.004 inches) and in one embodiment about 0.008 cm (0.003 inches). The resulting lamination generally has an average pore diameter within the range of from about 1 µm to about 100 µm, often within the range of from about 5 µm to about 60 µm, and in some applications, equivalent to ePTFE having an internodal distance within the range of from about 30 µm to about 60 µm.

In one embodiment, the resulting lamination generally has an average spacing between the edges of adjacent pores within the range of from about 50 µm to about 2,500 µm, often within the range of from about 50 µm to about 1000 µm, preferably within the range of from about 50 µm to about 300 µm, and in one embodiment within the range of from about 75 µm to about 150 µm. The resulting lamination generally has an open surface area of about 10% to about 50%, often about 10% to about 40%, preferably about 10% to 30% and in one embodiment about 35%. The optimum values for each of these physical parameters and the optimum relationship among these various physical parameters can be determined for any particular application and for any particular material or combination of materials through routine experimentation by one or ordinary skill in the art based on the disclosures herein.

As illustrated in the top plan view of **FIG. 16****,** the composite membrane barrier 15 of one embodiment has a plurality of "unbonded" windows, areas or pores 256. The pores 256 are suitable for cellular attachment and/or ingrowth. The windows 256 may be bounded by the frame 14 struts, and the cross-hatch or other wall pattern formed by the bonding layer 254, described further herein. In one embodiment, a regular window 256 pattern is produced in the bonding layer 254. The composite membrane barrier 15 generally allows cellular growth through and across the surface of the membrane barrier 15 within about two weeks after implantation, with endothelialization generally occurring within about one month.

In one embodiment, the outer layer 602, inner layer 604, bonding layer 606, and stent 608 are placed in a tube (not shown) and a balloon (not shown) is inserted inside of the lumen 612 formed by the inner layer 604. The balloon is inflated and heat is applied, as described in greater detail above, to cause the inner and outer layers 602, 602 to bond with bonding layer 606 and each other, thereby encapsulating stent 608 therebetween. As pressure and heat are applied to the composite lamination stent 600, the overlapping region 610 diminishes, thereby reducing the overall wall thickness of the composite laminate stent 600.

In another embodiment, the inner and outer layers 602, 604 of the composite lamination stent 600 are formed from tubes of ePTFE, or from a combination of tubes and sheets of ePTFE. For example, in one embodiment, the outer layer 602 comprises a tube of ePTFE, and the inner layer 604 comprises a sheet of ePTFE, such as that describe in greater detail above.

The stent 608 is self expanding, or expandable under force, such as by a balloon. In one embodiment, the stent 608 is formed from a single or multiple wire wrapped in a zig-zag or multiple zig-zag patterns, and in another embodiment, the stent is formed from a cut tube, such as a laser-cut tube of nickel titanium. Stents suitable for use in the composite lamination stent 600 are well known to those of skill in.the art.

Once assembled, in one embodiment, the cross section of the composite lamination stent 600 wall looks similar to the composite membrane stack 15 of **FIGS. 14** and **15****.** Window areas 256 (FIG. 16) in the wall of the composite lamination stent 600 allow for tissue ingrowth through the wall of the composite lamination stent 600, and for securement of the composite lamination stent 600 within the vessel in which the composite lamination stent 600 is inserted.

### II. ADDITIONAL COMPOSITE LAMINATION EXAMPLES - COMPOSITE LAMINATION SEPTAL DEFECT CLOSURE DEVICES

In one example, a composite lamination includes a septal defect closure device. Septal defect closure devices are well known to those of skill in the art as devices for sealing a variety of defects that may be found in the heart. Such defects include, for example, atrial septal defects (ASD), patent foramen ovale (PFO), and patent ductus arteriosis (PDA). An ASD is a defect in the septum of the heart between the left atrium and the right atrium. A PFO is a flap covering an opening in the septum that opens on occasion. A PDA is an opening between the aorta and the pulmonary artery. In one embodiment, a composite lamination is percutaneously provided to substantially close, or occlude any of these, or other defects.

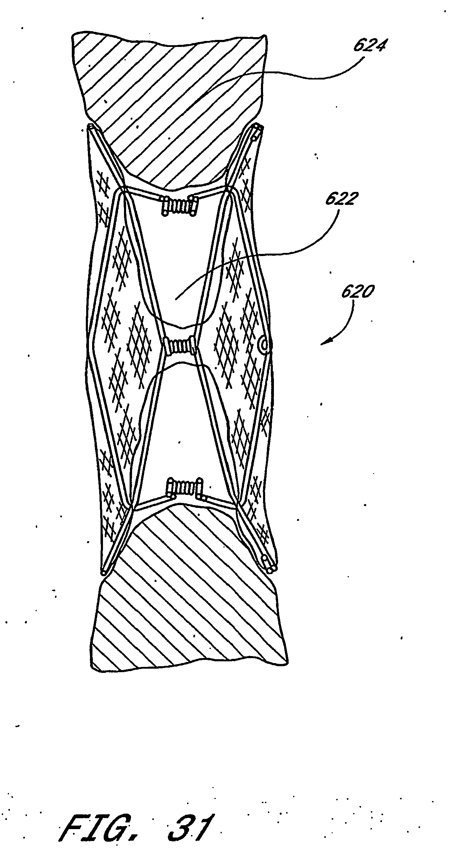

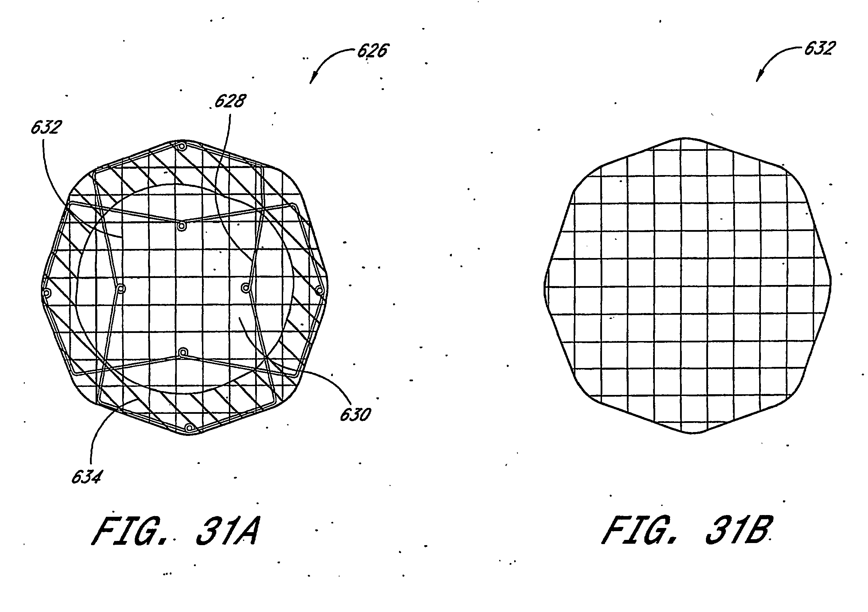

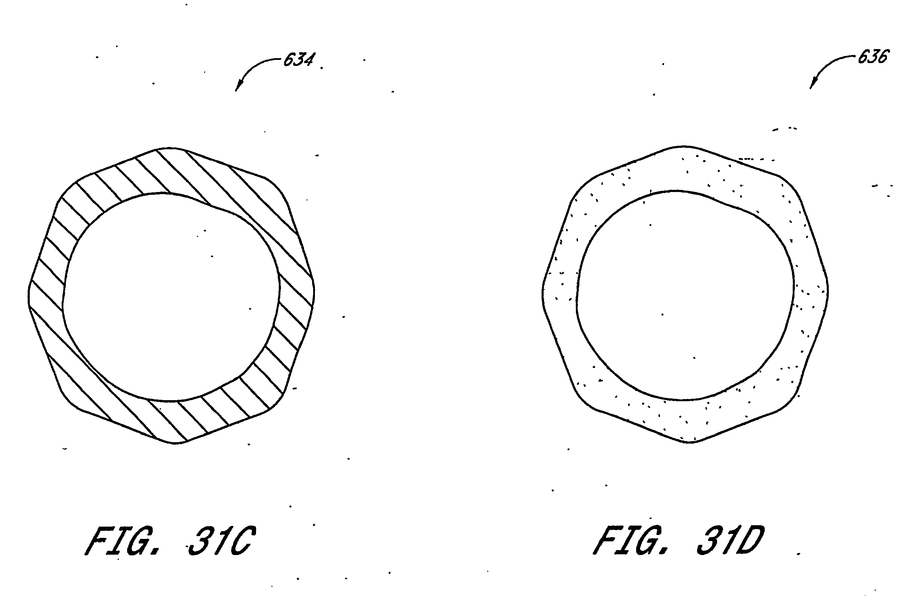

One example of a composite lamination septal defect closure device is illustrated in **FIG. 31****.** The illustrated composite lamination septal defect closure device 620 is shown expanded about a defect 622 in a septal wall 624. The composite lamination septal defect closure device 620 is formed from two occluder panels 626, as is well known to those of skill in the art, and as taught by Thill in U.S. Patent No. 6,551,344 and Gainor et al. in U.S. Patent No. 6,440,152. In one example, the occluder panel 626 includes at least one support frame 628 to which a membrane 630 is applied. In one embodiment, the membrane 630 includes ePTFE, and is attached to the support frame 628 according to any of the methods described in greater detail above.

In another example as illustrated in **FIG. 31A****,** the occluder panel 626 includes two overlapping, bow-tie-shaped support frames 628 to which an ePTFE lamination membrane 630 is applied. The illustrated example, membrane 630 is a lamination which includes a first layer 632, a second layer 634, and a bonding layer 636. In one example, as illustrated in **FIG. 31B****,** the first layer 632 has a circular shape suitable to cover the two support frames 628 of the occluder panel 626. The second layer 634 and bonding layer 636 each have a ring-like shape, as illustrated in **FIG. 31C** and **FIG. 31D****.** In one example, the support frames 628 are held between the first and second layers 632, 624 by the bonding layer 636. The first, second, and bonding layers 632, 634, 636, and the support frames 628 are secured together by using any of the methods described in greater detail above, such as by applying heat and pressure.

In one example the first layer 632 has a diameter larger than the diameter formed by the support frames 628 such that the edge of the first layer 632 wraps over the edges of the support frames 628, and is bonded to itself with the bonding layer 636. In this example the perimeter of the first layer 632 may have a notch, several notches, or other cut-out pattern to reduce or eliminate bunching up of the first layer 632 as it is folded over the support frames 628 and bonded to itself.

By bonding the frames 628 to the membrane 630 as described above, it is unnecessary to use other mechanical attachment apparatus, such as sutures. By avoiding the use of sutures, the membrane 630 maintains a stronger integrity, as the membrane 630 need not be punctured with holes through which sutures are passed.

In one example the membrane 630 functions as a baffle, barrier, or filter to blood flow across the defect 622. The membrane 630 also serves as a tissue ingrowth surface. It is advantageous to the physician to ascertain at the time of device implantation that flow across the defect 622 has been sealed. In one example I tissue growth into the membrane 630 is advantageous in that such ingrowth stabilizes the composite lamination septal defect closure device 620 and allows it to become a permanent part of the heart. In one embodiment, ePTFE is used as the membrane 630. In another example ePTFE is used to substantially obstruct the flow of blood across a septal defect 622.

In one example , the composite lamination septal defect closure device 620 is compressed into a low profile for delivery to a treatment site. In another example , the composite lamination septal defect closure device 620 does not inhibit expansion of self expanding or balloon expandable septal defect occluder frames 628. In another example, the composite lamination septal defect closure device 620 includes a membrane 630 that has a porosity so as to allow tissue ingrowth. In another example, ePTFE is used as first and/or second layers 632, 634 of the membrane 630 and substantially obstructs the flow of blood intra-op. In another example, the composite lamination septal defect closure device 620 provides superior and/or improved biocompatibility through the selection of ePTFE as the first and/or second layer 632, 634, and polyethylene as the bonding layer 636.

In one example, the first layer 632 and second layer 634 are similar or identical to the first and second membranes 250, 252, and outer and inner layers 602, 604 described in greater detail above. The bonding layer 636 may be an open mesh bonding layer similar or identical to bonding layer 254 and bonding layer 606 described above. In one example , the bonding layer 636 has an open surface area within the range of from about 20% to about 70%, often from about 30% to about 60%, and in some applications, from about 40% to about 50%, depending on the desired clinical performance.

The average pore diameter of the bonding layer 636 is preferably within the range of from about 0.01 cm (0.005 inches) to about 0.13 cm (0.050 inches). often from about 0.051 cm (0.020 inches) to about 0.10 cm (0.040 inches), and in some application from about 0.038 cm (0.015 inches) to about 0.076 cm (0.030 inches), depending on the desired clinical performance, and in one example, is about 0.051 cm (0.020 inches).

The thickness of the bonding layer 636 can be varied widely, and is generally within the range of from about 0.001 cm (0.0005 inches) to about 0.01 cm (0.005 inches), often from about 0.002 cm (0.0008 inches) to about 0.01 cm (0.004 inches), and in some applications from about 0.002 cm (0.0009 inches) to about 0.008 cm (0.003 inches) depending on the desired clinical performance. In one example, the bonding layer 636 has a thickness of about 0.003 cm (0.001 inches) to about 0.005 cm (0.002 inches). The optimum thickness can be determined for any particular application and for any particular material or combination of materials through routine experimentation by one or ordinary skill in the art based on the disclosure herein.

The first and second layers 632, 634 may have either the same or different thickness, and generally have a thickness within the range of from about 0.001 cm (0.0005 inches) to about 0.025 cm (0.10 inches), preferably from about 0.002 cm (0.0007 inches) to about 0.02 cm (0.008 inches), often from about 0.002 cm (0.0008 inches) to about 0.01 cm (0.005 inches), and in certain applications, from about 0.002 cm (0.0009 inches) to about 0.008 cm (0.003 inches). In one embodiment, the first and second layers 632, 634 each have a thickness on the order of from about 0.003 cm (0.001 inches) to about 0.005 cm (0.002 inches). The optimum thickness can be determined for any particular application and for any particular material or combination of materials through routine experimentation by one or ordinary skill in the art based on the disclosures herein.

The first and second layers 632, 634 may have either the same or different average pore diameters. The average pore diameter is often within the range of from about 1 µm to about 200 µm, preferably within the range of from about 5 µm to about 100 µm, and in some applications within the range of from about 10 µm to about 70 µm. The optimum average pore diameter or spacing can be determined for any particular application and for any particular material or combination of materials through routine experimentation by one or ordinary skill in the art based on the disclosures herein.

The first and second layers 632, 634 may have either the same or different average spacing between the edges of adjacent pores, and generally have an average internodal distance of from about 10 µm to about 100 µm, or exhibit equivalent cellular migration or ingrowth characteristics as layers having such internodular distances.

The resulting lamination of the occluder panel 626 of the composite lamination septal defect closure device 620 generally has a thickness within the range of from about 0.003 cm (0.001 inches) to about 0.025 cm (0.010 inches), often less than about 0.01 cm (0.005 inches), preferably less than about 0.01 cm (0.004 inches) and in one example about 0.008 cm (0.003 inches). The resulting lamination generally has an average pore diameter within the range of from about 1µm to about 100 µm, often within the range of from about 5µm to about 60 µm, and in some applications equivalent to ePTFE having an internodal distance within the range of from about 30 µm to about 60 µm.

In one example , the resulting lamination generally has an average spacing between the edges of adjacent pores within the range of from about 50 µm to about 2,500 µm, often within the range of from about 50 µm to about 1000 µm, preferably within the range of from about 50 µm to about 300 µm, and in one embodiment within the range of from about 75 µm to about 150 µm. The resulting lamination generally has an open surface area of about 10% to about 50%, often about 10% to about 40%, preferably about 10% to 30% and in one embodiment about 35%. The optimum values for each of these physical parameters and the optimum relationship among these various physical parameters can be determined for any particular application and for any particular material or combination of materials through routine experimentation by one or ordinary skill in the art based on the disclosures herein.

### III. ADDITIONAL COMPOSITE LAMINATION EXAMPLES COMPOSITE LAMINATION EMBOLIC PROTECTION DEVICES

In one example , a composite lamination includes an embolic protection device. Embolic protection devices are useful during angioplasty, stenting, aortic cross clamping, and other vessel intervention and manipulations that can cause the release of emboli from the vessel wall into the vascular system. Undesirable consequences may result from these emboli, such as stroke, no-reflow, trash foot, amputation, infarct, and others. Embolic protection devices are used to avoid these undesirable consequences as is well known to those of skill in the art.

In one example, an embolic protection device includes an occlusive device, and in another embodiment, an embolic protection device includes a filter. Occlusive devices are generally inflatable balloons or support structures covered with impermeable layers. Filters are generally flaccid meshes attached to an expandable support frame. Examples of such embolic protection device filters are described in U.S. Patent No. 6,391,044, and PCT Publication Nos. WO 00/16705, WO 99/23976, WO 00/67669, and US 6,325,815. The flaccid meshes include films with laser drilled holes, and woven or non-woven layers. In one embodiment, a filter includes a self expanding metal mesh with no support frame such as that described in PCT Publication NO. WO 96/01591.

In one example an embolic protection device is a distal protection device, and in another example it is a proximal protection device. A distal protection device crosses the treatment site, for example a lesion, after which it is deployed and positioned to capture emboli released during treatment. A proximal protection device is deployed proximal to a treatment site and generally relies upon reversal of flow in the vessel to remove emboli.

In one example , the flaccid mesh of the embolic protection device is designed to capture particles having a diameter in the range of about 10 microns to greater than 5,000 microns. In one example , the flaccid mesh has a pore size of about 100 microns. In another example , the flaccid mesh is used for cardiopulmonary bypass, and has a pore size of about 40 microns. In another example , the flaccid mesh has a pore size of 50 microns or smaller. In one example , the distal embolic protection device is a substantially occlusive device, and catheters are generally used to aspirate emboli in the vicinity of the substantially occlusive device.

In one example , the filter of an embolic protection device includes ePTFE, and separates emboli from blood flow. In one example , ePTFE is used to shed emboli formed on the ePTFE surface, and in another example , the ePTFE provides superior biocompatibility.

In one example , a composite lamination comprises a thin, flexible ePTFE membrane, and an embolic protection device, which includes an embolic protection device frame, as is well know to those of skill in the art. In one example , the ePTFE membrane is attached to the frame according to any of the methods described in greater detail above. In one example , the composite lamination is compressed into a low profile for delivery to a treatment site. In another example , the composite lamination does not inhibit expansion of self expanding or balloon expandable frames. In another example, the composite lamination includes a covering that has a porosity sufficient to filter embolic particles from blood. In one example , the composite lamination has a porosity sufficient to substantially occlude blood flow. In another example , the composite lamination provides superior and/or improved biocompatibility through the selection of ePTFE as the covering material, and polyethylene as the bonding layer.

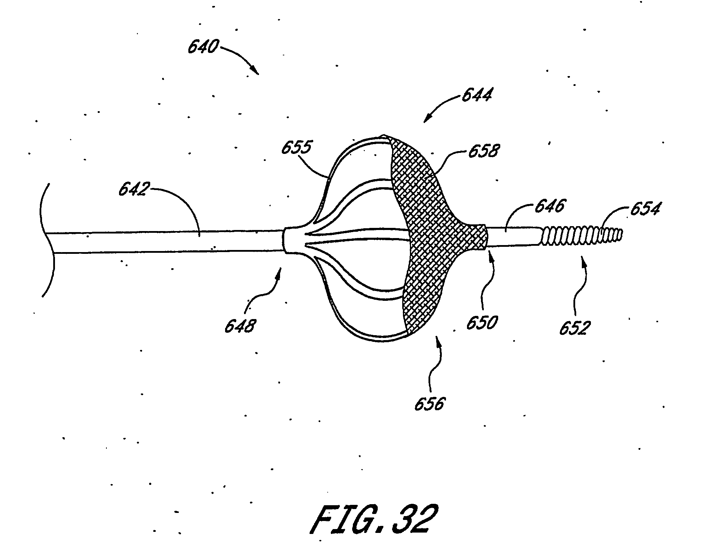

One example of a composite lamination embolic protection device is illustrated in **FIG. 32****.** In the illustrated example , the composite lamination embolic protection device 640 includes a host wire 642 and embolic collector 644 and a central wire 646. The host wire 642 is attached to the proximal end 648 of the embolic collector 644 and the central wire 646 is attached to the embolic collector 644 at the embolic collector's 644 distal end 650. In one example , the host wire 642 is 175 cm long and in another embodiment the host wire 642 is 320 cm long. The host wire 642 may include a lumen (not shown) through which the central wire 646 passes, such that the central wire 646 is axially movable with respect to the host wire 642. By moving the host wire 642 with respect to the central wire 646, the embolic collector 644 may be expanded from a small diameter configuration (not shown) to a large diameter, deployed configuration, as illustrated in FIG. 32. In another example , the portion of the central wire proximal to location 650 is omitted and the emboli collector is self expanding.

In one example , the distal end 652 of the central wire 646 is ground to a taper and includes a coil 654. In one example ., the coil 654 is welded at both of its ends to the central wire 646. Other methods of attaching the coil 654 to the central wire 646 are well known to those of skill in the art.

In one example , the embolic collector 644 is formed from a tube with longitudinal slots cut therethrough to form struts 655. In such example , when the central wire 646 is moved proximally with respect to the host wire 642, the embolic collector 644 expands, thereby extending struts 655 as shown in **FIG.32****.** The embolic collector 644 tube may be constructed from a variety of materials that are well known to those of skill in the art including, nickel titanium, and stainless steel. In one example such as the one illustrated in FIG. 32, the embolic collector 644 is mechanically deployed as a result of the movement of the central wire 646 with respect to the host wire 642. In other example , the embolic collector 644 is self-expandable or mechanically deployed by other methods as is well known to those of skill in the art.

In one example , the distal end 656 of the embolic collector 644 includes a lamination membrane 658. The lamination membrane 658 includes a first layer, a second layer and a bonding layer therebetween. The struts 655 of the embolic collector 644 are at least partially located between the first and second layers of the lamination membrane 658. The first layer, second layer, and bonding layer of the embolic collector 644 of **FIG. 32** is similar to that described above with respect to **FIGS. 13-16** and **FIGS. 30-31D****.** The lamination membrane 658 of the embolic collector 644 may be made by using any of the methods described above.

In one example , the first and second layers of the embolic collector 644 of the composite lamination embolic protection device 640 are similar or identical to the first and second membranes 250, 252, outer and inner layers 602, 604, and first and second layers 632, 634 described in greater detail above. The bonding layer of the embolic collector 644 of the composite lamination embolic protection device 640 may be an open mesh bonding layer similar or identical to bonding layer 254, bonding layer 606, and bonding layer 636 described above. In one example , the bonding layer has an open surface area within the range of from about 20% to about 70%, often from about 30% to about 60%, and in some applications, from about 40% to about 50%, depending on the desired clinical performance.

The average pore diameter of the bonding layer is preferably within the range of from about 0.01 cm (0.005 inches) to about 0.13 cm (0.050 inches), often from about 0.051 cm (0.020 inches) to about 0.10 cm (0.040 inches), and in some application from about 0.031 cm (0.015 inches) to about 0.076 cm (0.030 inches), depending on the desired clinical performance, and in one embodiment, is about 0.051 cm (0.020 inches).

The thickness of the bonding layer can be varied widely, and is generally within the range of from about 0.001 cm (0.0005 inches) to about 0.01 cm (0.005 inches), often from about 0.002 cm (0.0008 inches) to about 0.01 cm (0.004 inches) and in some applications from about 0.002 cm (0.0009 inches) to about 0.008 cm (0.003 inches) depending on the desired clinical performance. In one example, the bonding layer has a thickness of about 0.003 cm (0.001 inches) to about 0.005 cm (0.002 inches). The optimum thickness can be determined for any particular application and for any particular material or combination of materials through routine experimentation by one or ordinary skill in the art based on the disclosure herein.

The first and second layers may have either the same or different thicknesses, and generally have a thickness within the range of from about 0.001 cm (0.0005 inches) to about 0.025 cm (0.010 inches), preferably from about 0.002 cm (0.0007 inches) to about 0.02 cm (0.008 inches), often from about 0.002 cm (0.0008 inches) to about 0.01 cm (0.005 inches), and in certain applications, from about 0.002 cm (0.0009 inches) to about 0.008 cm (0.003 inches). In one example, the first and second layers each have a thickness on the order of from about 0.003 cm (0.001 inches) to about 0.005 cm (0.002 inches). The optimum thickness can be determined for any particular application and for any particular material or combination of materials through routine experimentation by one or ordinary skill in the art based on the disclosures herein.

The first and second layers may have either the same or different average pore diameters. The average pore diameter is often within the range of from about 1 µm to about 200 µm, preferably within the range of from about 5 µm to about 100 µm, and in some applications within the range of from about 10 µm to about 70 µm. The optimum average pore diameter or spacing can be determined for any particular application and for any particular material or combination of materials through routine experimentation by one or ordinary skill in the art based on the disclosures herein.

The first and second layers may have either the same or different average spacing between the edges of adjacent pores, and generally have an average internodal distance of from about 10 µm to about 100 µm, or exhibit equivalent cellular migration or ingrowth characteristics as layers having such internodular distances.

The resulting lamination of the embolic collector 644 of the composite lamination embolic protection device 640 generally has a thickness within the range of from about 0.003 cm (0.001 inches) to about 0.025 cm (0.010 inches), often less than about 0.01 cm (0.005 inches), preferably less than about 0.01 cm (0.004 inches) and in one example about 0.008 cm (0.003 inches). The resulting lamination generally has an average pore diameter within the range of from about 1 µm to about 100 µm, often within the range of from about 5µm to about 60µm, and in some applications, equivalent to ePTFE having an internodal distance within the range of from about 30 µm to about 60 µm.

In one example , the resulting lamination generally has an average spacing between the edges of adjacent pores within the range of from about 50 µm to about 2,500 µm, often within the range of from about 50 µm to about 1000 µm, preferably within the range of from about 50 µm to about 300 µm, and in one embodiment within the range of from about 75 µm to about 150 µm. The resulting lamination generally has an open surface area of about 10% to about 50%, often about 10% to about 40%, preferably about 10% to 30% and in one example about 35%. The optimum values for each of these physical parameters and the optimum relationship among these various physical parameters can be determined for any particular application and for any particular material or combination of materials through routine experimentation by one or ordinary skill in the art based on the disclosures herein.

While particular forms of the invention have been described, it will be apparent that various modifications can be made without departing from the scope of the invention. Accordingly, it is not intended that the invention be limited, except as by the appended claims.

## Claims

1. A laminated medical device (10), suitable for implantation within a medical patient, comprising:
a frame (14);
a first membrane, having first membrane pores (250);
a second membrane, having second membrane pores (252);
a mesh bonding layer (254), having a bonding layer open surface area and bonding layer pores, wherein the first membrane and second membrane are at least partially attached to the mesh bonding layer to form a composite membrane (15), wherein the composite membrane has a composite membrane open surface area in the range between about 10% and about 50% and **characterised in that** the device is a stent-graft and wherein the stent-graft is generally cylindrical and is adjustable from a first configuration having a reduced diameter to a second configuration having an expanded diameter, the stent-graft forming a conduit in the second configuration.

2. The laminated medical device of Claim 1, wherein the frame comprises a proximal hub, a distal hub, and struts spanning therebetween.

3. The laminated medical device of Claim 2, wherein the struts are formed by cutting slots out of a tube.

4. The laminated medical device of any of the preceding claims, wherein an element of the frame is disposed between the first membrane and the second membrane.

5. The laminated medical device of any of the preceding claims, wherein the composite membrane open surface area is sufficient to facilitate cellular ingrowth.

6. The laminated medical device of any of the preceding claims, wherein the composite membrane open surface area is sufficient to facilitate cellular attachment.

7. The laminated medical device of any of the preceding claims, wherein the composite membrane has an average thickness within the range of about 0.003 cm (0.001 inches) to about 0.025 cm (0.010 inches).

8. The laminated medical device of any of the preceding claims, wherein the first membrane comprises ePTFE.

9. The laminated medical device of any of the preceding claims, wherein the first membrane comprises a thickness in the range of from about 0.001 cm (0.0005 inches) to about 0.25 cm (0.10 inches).

10. The laminated medical device of any of the preceding claims, wherein the first membrane pores comprise an average pore diameter in the range of from about 1 µm to about 200 µm.

11. The laminated medical device of any of the preceding claims, wherein the first membrane comprises an internodal distance in the range of from about 10 µm to about 100 µm.

12. The laminated medical device of any of the preceding claims, wherein the bonding layer comprises polyethylene.

## Patentansprüche

1. Laminierte medizinische Vorrichtung (10), die zur Implantation in einen medizinischen Patienten geeignet ist, wobei die Vorrichtung aufweist:
einen Rahmen (14),
eine erste Membran mit ersten Membranporen (250),
eine zweite Membran mit zweiten Membranporen (252),
eine Netzbindungsschicht (254) mit einer offenen Oberfläche der Bindungsschicht und Bindungsschichtporen, wobei die erste Membran und die zweite Membran wenigstens teilweise an die Netzbindungsschicht gebunden sind, um eine Verbundmembran (15) zu bilden, wobei die Verbundmembran eine offene Oberfläche der Verbundmembran im Bereich zwischen etwa 10% und etwa 50% hat, und **dadurch gekennzeichnet, daß** die Vorrichtung ein Stenttransplantat ist und wobei das Stenttransplantat im allgemeinen zylindrisch ist und von einer ersten Ausgestaltung mit einem reduzierten Durchmesser auf eine zweite Ausgestaltung mit einem erweiterten Durchmesser einstellbar ist, wobei das Stenttransplantat in der zweiten Ausgestaltung eine Leitung bzw. einen Kanal bildet.

2. Laminierte medizinische Vorrichtung nach Anspruch 1, wobei der Rahmen eine proximale Nabe, eine distale Nabe und sich dazwischen erstreckende Streben aufweist.

3. Laminierte medizinische Vorrichtung nach Anspruch 2, wobei die Streben gebildet werden, indem man Schlitze aus einem Rohr ausschneidet.

4. Laminierte medizinische Vorrichtung nach einem der vorangegangenen Ansprüche, wobei ein Element des Rahmens zwischen der ersten Membran und der zweiten Membran angeordnet ist.

5. Laminierte medizinische Vorrichtung nach einem der vorangegangenen Ansprüche, wobei die offene Oberfläche der Verbundmembran ausreichend ist, um das Einwachsen von Zellen zu erleichtern.

6. Laminierte medizinische Vorrichtung nach einem der vorangegangenen Ansprüche, wobei die offene Oberfläche der Verbundmembran ausreichend ist, um die Anhaftung von Zellen zu erleichtern.

7. Laminierte medizinische Vorrichtung nach einem der vorangegangenen Ansprüche, wobei die Verbundmembran eine mittlere Dicke im Bereich von etwa 0,003 cm (0,001 Zoll) bis etwa 0,025 cm (0,010 Zoll) hat.

8. Laminierte medizinische Vorrichtung nach einem der vorangegangenen Ansprüche, wobei die erste Membran ePTFE beinhaltet.

9. Laminierte medizinische Vorrichtung nach einem der vorangegangenen Ansprüche, wobei die erste Membran eine Dicke im Bereich von etwa 0,001 cm (0,0005 Zoll) bis etwa 0,25 cm (0,10 Zoll) hat.

10. Laminierte medizinische Vorrichtung nach einem der vorangegangenen Ansprüche, wobei die ersten Membranporen einen mittleren Porendurchmesser im Bereich von etwa 1 µm bis etwa 200 µm haben.

11. Laminierte medizinische Vorrichtung nach einem der vorangegangenen Ansprüche, wobei die erste Membran einen internodalen Abstand im Bereich von etwa 10 µm bis etwa 100 µm hat.

12. Laminierte medizinische Vorrichtung nach einem der vorangegangenen Ansprüche, wobei die Bindungsschicht Polyethylen beinhaltet.

## Revendications

1. Dispositif médical feuilleté (10), apte à une implantation chez un patient médical, comportant :
une ossature (14) ;
une première membrane ayant des pores (250) de première membrane ;
une seconde membrane ayant des pores (252) de seconde membrane ;
une couche maillée (254) de liaison ayant une surface spécifique ouverte de couche de liaison et des pores de couche de liaison, dans lequel la première membrane et la seconde membrane sont attachées au moins partiellement à la couche maillée de liaison pour former une membrane composite (15), dans lequel la membrane composite a une surface spécifique ouverte de membrane composite dans la plage comprise entre environ 10 % et environ 50 %, et **caractérisé en ce que** le dispositif est un extenseur-greffe et l'extenseur-greffe est globalement cylindrique et est réglable d'une première configuration ayant un diamètre réduit à une seconde configuration ayant un diamètre expansé, l'extenseur-greffe formant un conduit dans la seconde configuration.

2. Dispositif médical feuilleté selon la revendication 1, dans lequel l'ossature comporte un moyeu proximal, un moyeu distal et des entretoises s'étendant entre eux.

3. Dispositif médical feuilleté selon la revendication 2, dans lequel les entretoises sont formées en découpant des fentes dans un tube.

4. Dispositif médical feuilleté selon l'une quelconque des revendications précédentes, dans lequel un élément de l'ossature est disposé entre la première membrane et la seconde membrane.

5. Dispositif médical feuilleté selon l'une quelconque des revendications précédentes, dans lequel la surface spécifique ouverte de la membrane composite est suffisante pour faciliter une croissance cellulaire pénétrante.

6. Dispositif médical feuilleté selon l'une quelconque des revendications précédentes, dans lequel la surface spécifique ouverte de la membrane composite est suffisante pour faciliter une attache cellulaire.

7. Dispositif médical feuilleté selon l'une quelconque des revendications précédentes, dans lequel la membrane composite a une épaisseur moyenne dans la plage d'environ 0,003 cm (0,001 in) à environ 0,025 cm (0,010 in).

8. Dispositif médical feuilleté selon l'une quelconque des revendications précédentes, dans lequel la première membrane comprend du ePTFE.

9. Dispositif médical feuilleté selon l'une quelconque des revendications précédentes, dans lequel la première membrane a une épaisseur dans la plage d'environ 0,001 cm (0,0005 in) à environ 0,25 cm (0,10 in).

10. Dispositif médical feuilleté selon l'une quelconque des revendications précédentes, dans lequel les pores de la première membrane ont un diamètre moyen de pores dans la plage d'environ 1 µm à environ 200 µm.

11. Dispositif médical feuilleté selon l'une quelconque des revendications précédentes, dans lequel la première membrane présente une distance internodale dans la plage d'environ 10 µm à environ 100 µm.

12. Dispositif médical feuilleté selon l'une quelconque des revendications précédentes, dans lequel la couche de liaison comprend du polyéthylène.
